# EUROPEAN PATENT APPLICATION

(11) **EP 3 778 893 A1**
(43) Date of publication of application: **17.02.2021**
(21) Application number: 19785602.4
(22) Date of filing: 10.04.2019
(51) Int. Cl.: C12N 15/113, A61K 31/713, A61P 35/00

(54) **METHOD FOR ACTIVATING P21 GENE EXPRESSION**

(30) Priority: 10.04.2018 CN 201810316672
(71) Applicant: Ractigen Therapeutics, Nantong, Jiangsu 226400 (CN)
(72) Inventor: LI, Longcheng, Nantong, Jiangsu 226400 (CN); KANG, Moorim, Nantong, Jiangsu 226400 (CN); WU, Jiancheng, Nantong, Jiangsu 226400 (CN)
(74) Representative: Høiberg P/S
(86) International application number: PCT/CN2019/082126
(87) International publication number: WO 2019/196883

(57) **Abstract**

The present invention provides a saRNA for activating or upregulating human p21 expression, the saRNA comprises a sense oligonucleotide strand and an antisense oligonucleotide strand, and the sense nucleic acid strand or the antisense nucleic acid strand has more than 75% homology with any continuous fragment of 16 to 35 nucleotides in length in the target gene sequence of a human p21 promoter, wherein the target nucleic acid sequence of the human p21 promoter is selected from a group consisting of SEQ ID NOs: 5, 6, 7, 8, 9, 10, 11, and 12.

## Description

### FIELD OF THE INVENTION

The present invention relates to the field of molecular biology, and in particular to up-regulating gene expression with a double-stranded small RNA targeting a gene promoter.

### BACKGROUND OF THE INVENTION

Double-stranded small nucleic acid molecules including chemically- synthesized oligoribonucleotides (such as small activating RNA (saRNA)) and naturally occurring oligoribonucleotides (such as micro ribonucleic acid (miRNA)) have been proven to be capable of targeting regulatory sequences (such as promoter sequences) of protein-coding genes in a sequence-specific manner to up-regulate gene expression at the transcriptional and epigenetic level, a phenomenon known as RNA activation (RNAa) (Li, Okino et al. (2006) Proc Natl Acad Sci U S A 103:17337-17342; Janowski, Younger et al. (2007) Nat Chem Biol 3:166-173; Place, Li et al. (2008) Proc Natl Acad Sci U S A 105:1608-1613; Huang, Place et al. (2012) Nucleic Acids Res 40:1695-1707; Li (2017) Adv Exp Med Biol 983:1-20). Studies have shown that RNA-a is an endogenous molecular mechanism evolutionarily conserved from caenorhabditis elegans to the human being (Huang, Qin et al. (2010) PLoS One 5:e8848; Seth, Shirayama et al. (2013) Dev Cell 27:656-663; Turner, Jiao et al. (2014) Cell Cycle 13:772-781).

Safe strategies to selectively enhance gene and/or protein production remain a challenge in gene therapy. Viral-based systems have inherent drawbacks including adverse effects on host genome integrity and immunological consequences. RNAa, with the advantages of being able to activate endogenous genes without the risk of altering the genome, represents a new strategy to stimulate target gene expression.

Cyclin-dependent kinase (CDK) inhibitor p21^{WAF1/CIP1} (p21) is a mediator of several anti-growth pathways and considered a potent tumor suppressor gene in cancer cells (Harper, Adami et al. (1993) Cell 75:805-816). In fact, overexpression of p21 by ectopic vectors or stimulation of endogenous transcription inhibits tumor growth both in vitro and in vivo (Harper, Adami et al. (1993) Cell 75:805-816; Eastham, Hall et al. (1995) Cancer Res 55:5151-5155; Wu, Bellas et al. (1998) J Exp Med 187:1671-1679; Harrington, Spitzweg et al. (2001) J Urol 166:1220-1233). As such, selective activation of p21 can possess therapeutic application for regulating cell growth and treatment of disease (e.g. cancer).

### SUMMARY OF THE INVENTION

The present invention provides a saRNA, wherein one strand of the saRNA has at least 75% homology or complementarity with any continuous fragment of 16 to 35 nucleotides in length in a nucleic acid sequence selected from the group consisting of SEQ ID NOs: 5, 6, 7, 8, 9, 10, 11, and 12, and wherein the saRNA activates or upregulates the expression of p21 by targeting the sequence of a human p21 promoter.

In certain embodiments, a target gene sequence of the human p21 is selected from a group consisting of SEQ ID NO: 5-12, wherein the sequence of the human p21 promoter is from -893 bp to -801 bp (SEQ ID NO: 5), -717 bp to -632 bp (SEQ ID NO: 6), -585 bp to - 551 bp (SEQ ID NO: 7), -554 bp to -504 bp (SEQ ID NO: 8), -514 bp to -485 bp (SEQ ID NO: 9), -442 bp to -405 bp (SEQ ID NO: 10), -352 bp to -313 bp (SEQ ID NO: 11), or -325 bp to -260 bp (SEQ ID NO: 12) upstream of the transcription start site (TSS) respectively.

In certain embodiments, the saRNA comprises a sense nucleic acid strand and an antisense nucleic acid strand. The sense nucleic acid strand and the antisense nucleic acid strand can contain complementary regions capable of forming a double-stranded nucleic acid structure, and the sense nucleic acid strand or the antisense nucleic acid strand has more than 75%, more than 80%, more than 90%, more than 95%, more than 99%, or 100% homology with any continuous fragment of 16 to 35 nucleotides in length in a sequence of a human p21 promoter.

In certain embodiments, the sense nucleic acid strand and the antisense nucleic acid strand are on two different nucleic acid strands; or the sense nucleic acid strand and the antisense nucleic acid strand are on the same nucleic acid strand, forming a hairpin single-stranded nucleic acid molecule, wherein the complementary regions of the sense nucleic acid strand and the antisense nucleic acid strand form a double-stranded nucleic acid structure.

In certain embodiments, at least one strand of the saRNA has a 3' overhang of 0 to 6 nucleotides in length; or both strands of the saRNA have a 3' overhang of 2 to 3 nucleotides in length.

In certain embodiments, the sense nucleic acid strand or the antisense nucleic acid strand has 16 to 35 nucleotides, such as 16, 17, 18, 19, 20, 21, 22, 23, 24, 25, 26, 27, 28, 29, 30, 31, 32, 33, 34, or 35 nucleotides, in length.

In certain embodiments, the sense nucleic acid strand or the antisense nucleic acid strand has at least 75%, such as 80%, 85%, 90%, 95%, 99%, or 100%, homology with a nucleic acid sequence selected from the group consisting of SEQ ID NOs: 13-30, 35-46, 59-62, 67-74, 77-80, 85-96, 103-108, 111-118, 121-132, 139-140, 147-180, 185-186, 189-190, 195-198, 201-202, 209-212, 215-218, 225-240, 243-246, 249-258, 261-262, 265-270, 275-280, 283-300, 303-308, 317-320, 323-324, 329-348, 351-352, 357-358, 361-366, 371-392, 399-400, 405-412, 415-416, 419-424, 429-432, 439-442, 447-450, 453-458, and 463-468.

In certain embodiments, the nucleotide sequence of the sense nucleic acid strand or the antisense nucleic acid strand is selected from the group consisting of SEQ ID NOs: 13-30, 35-46, 59-62, 67-74, 77-80, 85-96, 103-108, 111-118, 121-132, 139-140, 147-180, 185-186, 189-190, 195-198, 201-202, 209-212, 215-218, 225-240, 243-246, 249-258, 261-262, 265-270, 275-280, 283-300, 303-308, 317-320, 323-324, 329-348, 351-352, 357-358, 361-366, 371-392, 399-400, 405-412, 415-416, 419-424, 429-432, 439-442, 447-450, 453-458, and 463-468.

The present invention further provides a method for preparing the saRNA as described by any one of the aforementioned examples, wherein the method comprises the following steps: 1) selecting a sequence containing 19 bases as a target site by using the sequence of a promoter of a target gene as a template; 2) synthesizing a RNA sequence having more than 75% homology with the sequence of the target site obtained in step 1) to obtain a sense oligonucleotide strand; 3) synthesizing a sequence complementary with the sense oligonucleotide strand obtained in step 2); and 4) mixing the sense oligonucleotide strand obtained in step 2) and an antisense oligonucleotide strand obtained in step 3) in RNA annealing buffer at a molar ratio of 1:1, heating the mixture, and then naturally cooling the mixture to room temperature, so that a double-stranded saRNA is obtained, wherein the nucleic acid sequence of the human p21 promoter is selected from a group consisting of SEQ ID NOs: 5, 6, 7, 8, 9, 10, 11, and 12.

In certain embodiments, at least one nucleotide of the saRNA is a chemically modified nucleotide, and the chemical modification is at least one of the following modifications:
(1) modification of a phosphodiester bond connecting nucleotides in the nucleotide sequence of the saRNA;
(2) modification of 2'-OH of ribose in the nucleotide sequence of the saRNA;
(3) modification of a base in the nucleotide sequence of the saRNA; or
(4) at least one nucleotide in the nucleotide sequence of the small activating nucleic acid molecule being a locked nucleic acid.

In certain embodiments, the expression of p21 is activated or upregulated by at least 10%, such as more than 15%, 20%, 30%, 40%, 50%, 80%, 100%, or 200%.

The present invention further provides a use of the saRNA in the preparation of a formulation for activating or upregulating the expression of p21 in a cell. The saRNA is introduced into the cell directly, or is produced in the cell after a nucleotide sequence encoding the saRNA is introduced into the cell. The cell is a mammal cell, preferably a human cell, and more preferably a human tumor cell. The human cell can be an isolated human cell line or can be present in a human body.

In certain embodiments, the human body is a patient with a tumor caused by insufficient p21 protein expression, wherein administration of an effective amount of the small activating nucleic acid molecule can treat the tumor, and the tumor is preferably a bladder cancer, a prostate cancer, a hepatocellular carcinoma, or a colorectal cancer.

In another aspect, the present invention further provides an isolated p21 saRNA target site, wherein the target site is any continuous 16-35 nucleotide sequence selected from the group consisting of SEQ ID NOs. 5-12.

In yet another aspect, the present invention discloses a method for activating or upregulating the expression of human p21 in a cell, wherein the method comprises administrating the saRNA as described in any one of the aforementioned embodiments to a subject or a cell. The saRNA can be introduced into the cell directly, or can be produced in the cell after a nucleotide sequence encoding the saRNA is introduced into the cell. The cell is a mammalian cell, preferably a human cell, more preferably a human tumor cell, and most preferably a bladder cancer cell, a prostate cancer cell, a hepatocellular carcinoma cell, or a colorectal cancer cell.

The present invention further discloses a composition containing the aforementioned saRNA and a pharmaceutically acceptable carrier. The pharmaceutically acceptable carrier is a liposome, a macromolecular polymer, or a polypeptide.

The present invention further discloses a use of the saRNA or the composition as described above in the preparation of a formulation for activating or upregulating p21 expression. Preferably a use of the saRNA or the composition in the preparation of a formulation is for treating a tumor or a benign proliferative lesion. Preferably, the tumor is a bladder cancer, a prostate cancer, a hepatocellular carcinoma, or a colorectal cancer.

### BRIEF DESCRIPTION OF THE DRAWINGS

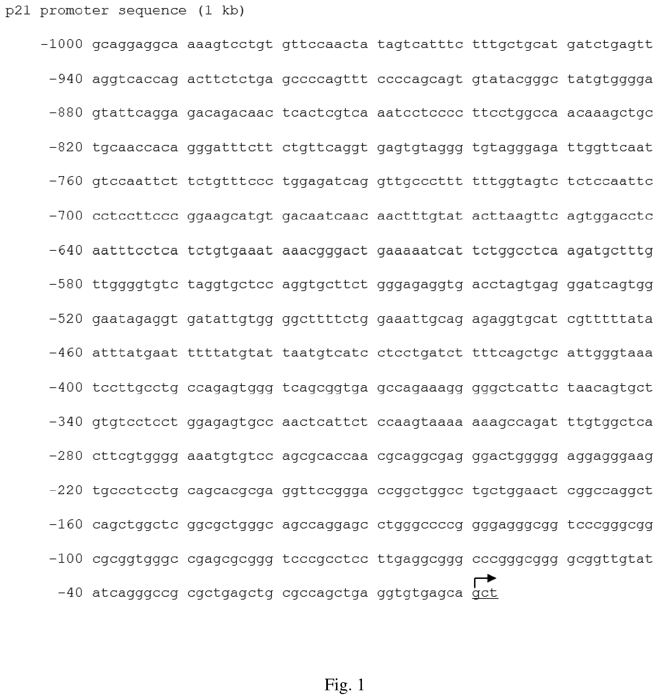

Fig. 1 shows the sequence of the p21 gene promoter, from -1000 bp upstream of the transcription start site (TSS) to +3 bp downstream of the TSS (SEQ ID NO: 469). The TSS is represented by a bent arrow.

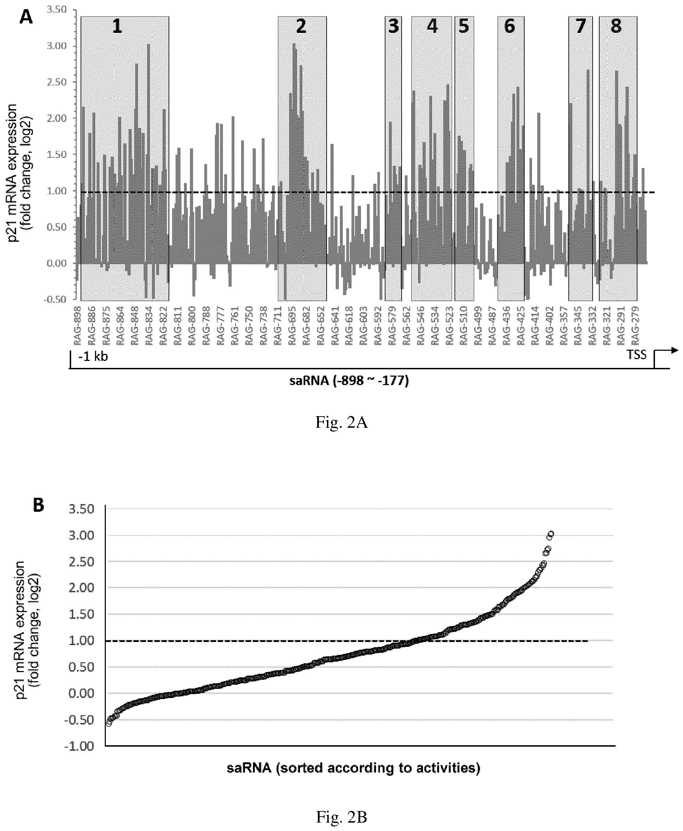

Figs. 2A and 2B show saRNA hotspot regions in the p21 promoter as revealed by screening. According to the sequence of the p21 promoter shown in Fig. 1, four hundred and thirty-nine (439) double-stranded RNA molecules were designed, chemically synthesized, and each double-stranded RNA molecule was transfected into PC3 human prostate cancer cells. 72 hours after transfection, the mRNA levels of p21 were determined by using QuantiGene 2.0 assay. Fig. 2A shows the fold change (Y-axis) of p21 mRNA levels caused by each of the 439 double-stranded RNA molecules (X-axis) relative to a control treatment (Mock). The double-stranded RNA molecules on the X-axis were sorted according to their positions relative to the TSS of p21 (from the most upstream RAG-898 to the most downstream RAG-177). Eight hotspot regions were identified and shown (grayish rectangular boxes). FIG. 2B shows the data of FIG. 2A by sorting the double-stranded RNA molecules by their activity in activating p21 mRNA expression in ascending order. The dotted lines in Fig. 2A and Fig. 2B represent the two-fold induction.

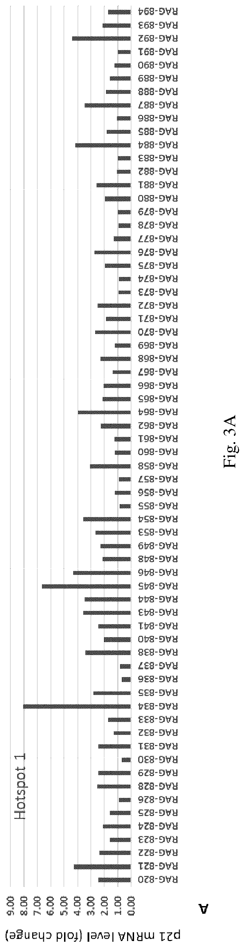

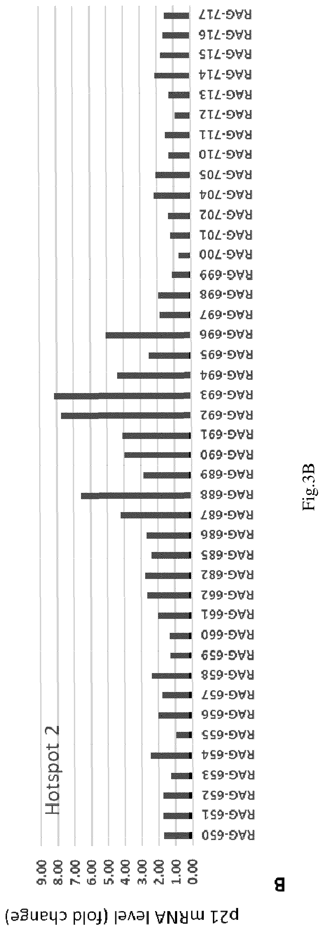

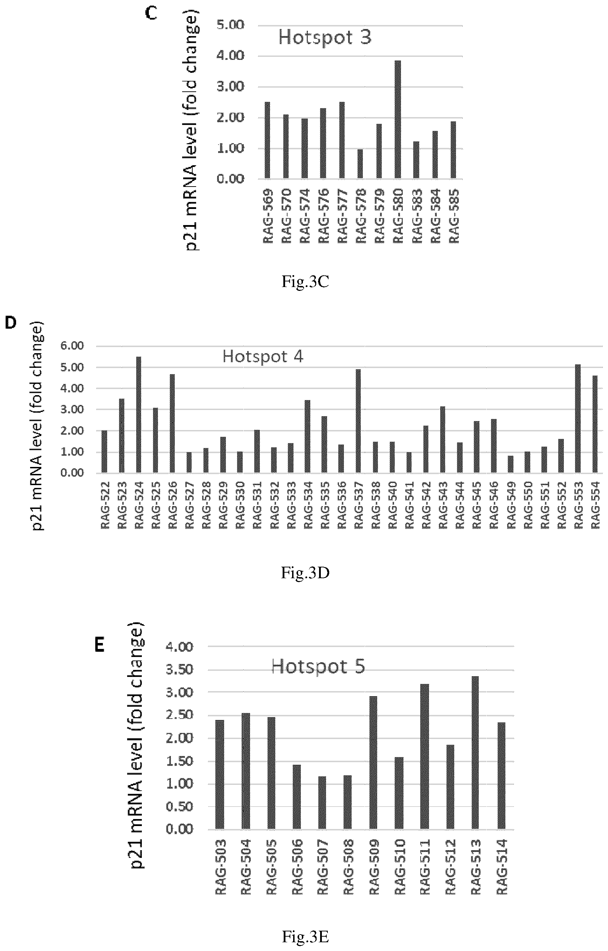

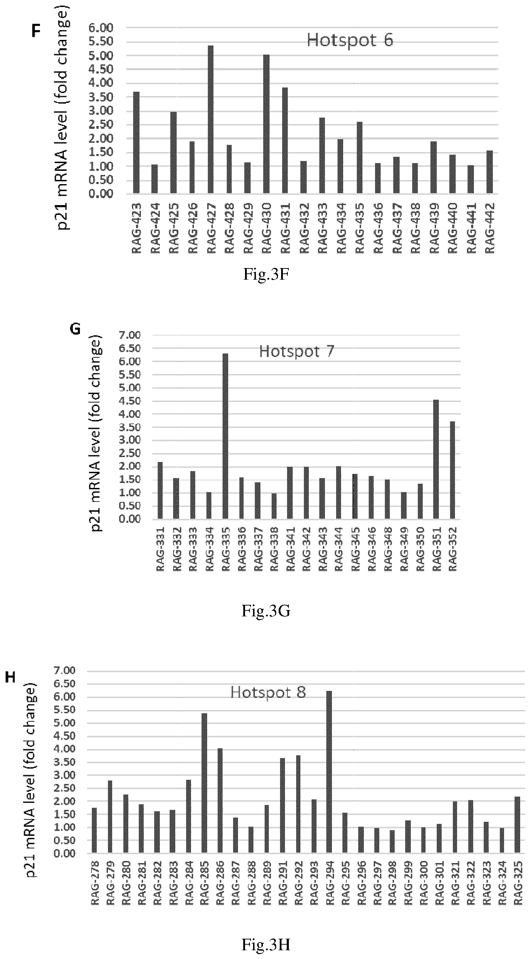

Figs. 3A-3H show the activating effects of the double-stranded RNA molecules targeting the hotspot regions 1 to 8 on the p21 promoter (FIG. 3A: hotspot region 1; FIG. 3B: hotspot region 2; FIG. 3C: hotspot region 3; FIG. 3D: hotspot region 4; FIG. 3E: hotspot region 5; FIG. 3F: hotspot region 6; FIG. 3G: hotspot region 7; FIG. 3H: hotspot region 8).

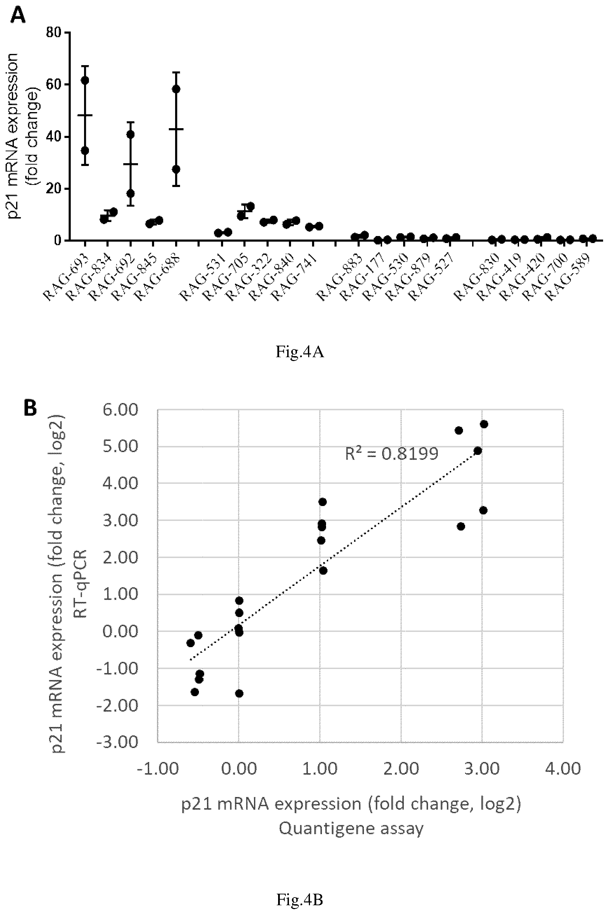

Figs. 4A and 4B show the mRNA levels of p21 assessed by RT-qPCR for verifying the result obtained from QuantiGene 2.0 assay. FIG. 4A shows the p21 mRNA level determined by RT-qPCR. The 439 double-stranded RNA molecules were divided into four groups according to their activities in inducing p21 mRNA expression from the highest to the lowest, and 5 double-stranded RNA molecules were randomly selected from each group and individually transfected into PC3 cells at a concentration of 10 nM. 72 hours after transfection, total cellular RNA was extracted from the transfected cells and reverse transcribed into cDNA, which was amplified by RT-qPCR to determine p21 mRNA level. FIG. 4B shows the correlation of relative p21 mRNA level induced by the double-stranded RNA molecules as determined by QuantiGene 2.0 (X-axis) and by RT-qPCR (Y-axis).

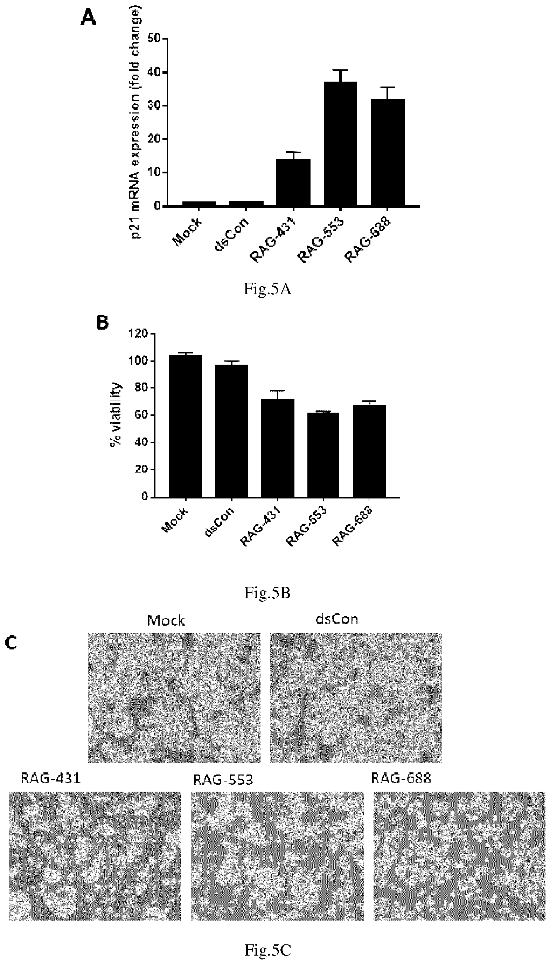

Figs. 5A-5C show the effect of the saRNAs in inducing the mRNA expression of p21 and suppressing the proliferation of KU-7 cells. KU-7 cells were transfected with each of three saRNAs (RAG-431, RAG-553, or RAG-688) at a concentration of 10 nM for 72 hours. FIG. 5A shows the mRNA expression levels of p21 determined by RT-qPCR. FIG. 5B shows the viability of saRNA treated cells as evaluated by the CCK-8 assay and plotted as percentages relative to the cell viability for control treatment (Mock). FIG. 5C shows representative phase-contrast cell images (100 ×) at the end of transfection.

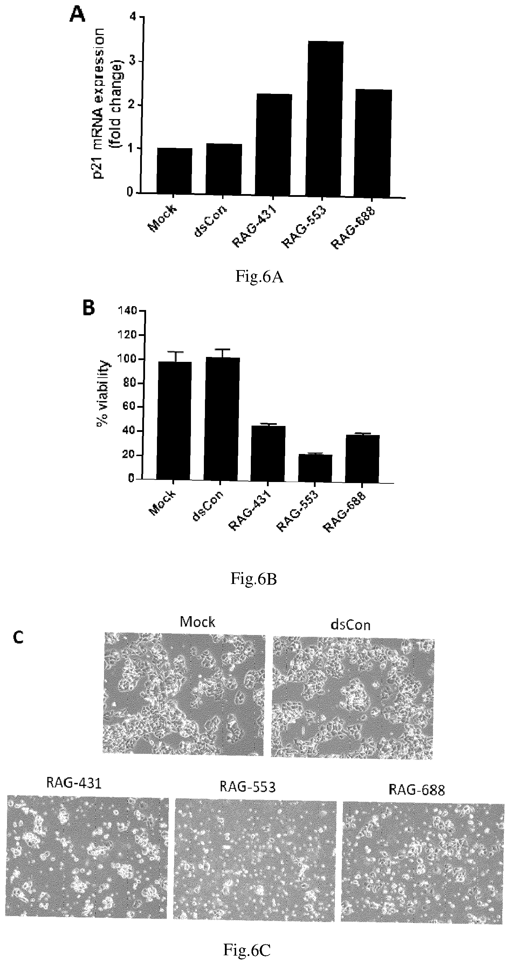

Figs. 6A-6C show the effect of the saRNAs in inducing the mRNA expression of p21 and suppressing the proliferation of HCT116 cells. HCT116 cells were transfected with each of the three saRNAs (RAG-431, RAG-553, or RAG-688) at a concentration of 10 nM for 72 hours. FIG. 6A shows the mRNA expression levels of p21 determined by RT-qPCR. FIG. 6B shows the cell viability as evaluated by the CCK-8 assay and plotted as percentages relative to the cell viability for control treatment (Mock). FIG. 6C shows representative phase-contrast cell images (100 ×) at the end of transfection.

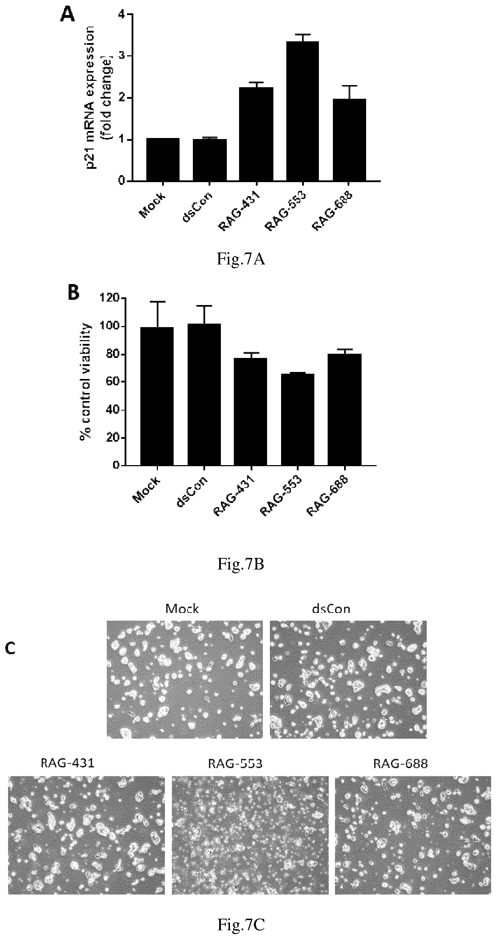

Figs. 7A-7C show the effect of the saRNAs in inducing the mRNA expression of p21 and suppressing the proliferation of HepG2 cells. HepG2 cells were transfected with each of the three saRNAs (RAG-431, RAG-553, or RAG-688) at a concentration of 10 nM for 72 hours. FIG. 7A shows the mRNA expression levels of p21 determined by RT-qPCR. FIG. 7B shows the cell viability as evaluated by the CCK-8 assay and plotted as percentages relative to the cell viability for control treatment (Mock). FIG. 7C shows representative phase-contrast cell images (100 ×) at the end of transfection.

### DETAILED DESCRIPTION OF THE INVENTION

The present invention is further described hereinafter by specific description.

Unless otherwise defined, all the technological and scientific terms used herein have the same meanings as those generally understood by those of ordinary skill in the art covering the present invention.

In the present application, singular forms, such as "a" and "this", include plural objects, unless otherwise specified clearly in the context.

### Definition

The term "complementary" as used herein refers to the capability of forming base pairs between two oligonucleotide strands. The base pairs are generally formed by hydrogen bonds between nucleotide units in the antiparallel oligonucleotide strands. The bases of the complementary oligonucleotide strands can be paired in the Watson-Crick manner (such as A to T, A to U, and C to G) or in any other manner allowing the formation of a duplex (such as Hoogsteen or reverse Hoogsteen base pairing). "100% pairing" or "complete complementarity" refers to 100% complementarity, that is, all the nucleotide units of the two strands are bound by hydrogen bonds.

"Complete complementarity" or "100% pairing" means that each nucleotide unit from the first oligonucleotide strand can form a hydrogen bond with the second oligonucleotide strand in the double-stranded region of the double-stranded oligonucleotide molecule, with no base pair being "mispaired". "Incomplete complementarity" means that not all the nucleotide units of the two strands are bound with each other by hydrogen bonds. For example, for two oligonucleotide strands each of 20 nucleotides in length in the double-stranded region, if only two base pairs in this double-stranded region can be formed through hydrogen bonds, the oligonucleotide strands have a complementarity of 10%. In the same example, if 18 base pairs in this double-stranded region can be formed through hydrogen bonds, the oligonucleotide strands have a complementarity of 90%. "Substantial complementarity" refers to more than about 79%, about 80%, about 85%, about 90%, or about 95% complementarity.

The term "oligonucleotide" as used herein refers to polymers of nucleotides, and includes, but is not limited to, single-stranded or double-stranded molecules of DNA, RNA, DNA/RNA hybrid, oligonucleotide strands containing regularly and irregularly alternating deoxyribosyl portions and ribosyl portions, as well as modified and naturally or unnaturally existing frameworks for such oligonucleotides.

The term "oligoribonucleotide" as used herein refers to an oligonucleotide containing two or more modified or unmodified ribonucleotides and/or analogues thereof.

The terms "oligonucleotide strand" and "oligonucleotide sequence" as used herein can be used interchangeably, referring to a generic term for short nucleotide sequences having less than 50 bases (the nucleic acid can be deoxyribonucleic acid (DNA) or ribonucleic acid (RNA)). In the present invention, the length of an oligonucleotide strand can be any length from 17 to 30 nucleotides.

The term "gene" as used herein refers to all nucleotide sequences required to encode a polypeptide chain or to transcribe a functional RNA. "Gene" can be an endogenous or fully or partially recombinant gene for a host cell (for example, because an exogenous oligonucleotide and a coding sequence for coding a promoter are introduced into a host cell, or a heterogeneous promoter adjacent to an endogenous coding sequence is introduced into a host cell). For example, the term "gene" includes a nucleic acid sequence composed of exons and introns. Protein-coding sequences are, for example, sequences contained within exons in an open reading frame between an initiation codon and a termination codon, and as used herein, "gene" can comprise a gene regulatory sequence, such as a promoter, an enhancer, and all other sequences known in the art for controlling the transcription, expression or activity of another gene, no matter whether the gene contains a coding sequence or a non-coding sequence. In one case, for example, "gene" can be used to describe a functional nucleic acid containing a regulatory sequence such as a promoter or an enhancer. The expression of a recombinant gene can be controlled by one or more types of heterogenous regulatory sequences.

The term "target gene" as used herein can refer to nucleic acid sequences, transgenes, viral or bacterial sequences, chromosomes or extrachromosomal genes that are naturally present in organisms, and/or can be transiently or stably transfected or incorporated into cells and/or chromatins thereof. The target gene can be a protein-coding gene or a non-protein-coding gene (such as microRNA gene and long non-coding RNA gene). The target gene generally contains a promoter sequence, and the positive regulation for the target gene can be achieved by designing a saRNA having sequence homology with the promoter sequence, characterized as the upregulation of expression of the target gene. "Sequence of a target gene promoter" refers to a non-coding sequence of the target gene, and the reference of the sequence of a target gene promoter in the phrase "complementary with the sequence of a target gene promoter" of the present invention means a coding strand of the sequence, also known as a non-template strand, i.e. a nucleic acid sequence having the same sequence as the coding sequence of the gene. "Target sequence" refers to a sequence fragment in the target gene promoter sequence, which is homologous or complementary with a sense oligonucleotide strand or an antisense oligonucleotide strand of a saRNA.

As used herein, the terms "sense strand" and "sense oligonucleotide strand" can be used interchangeably, and the sense oligonucleotide strand of a saRNA refers to a first ribonucleic acid strand having homology with the coding strand of the promoter sequence of the target gene in the saRNA duplex.

As used herein, the terms "antisense strand" and "antisense oligonucleotide strand" can be used interchangeably, and the antisense oligonucleotide strand of a saRNA refers to a second ribonucleic acid strand complementary with the sense oligonucleotide strand in the saRNA duplex.

The term "coding strand" as used herein refers to a DNA strand in the target gene which cannot be used for transcription, and the nucleotide sequence of this strand is the same as that of RNA produced from transcription (in the RNA, T in DNA is replaced by U). The coding strand of the double-stranded DNA sequence of the target gene promoter described in the present invention refers to a promoter sequence on the same DNA strand as the DNA coding strand of the target gene.

The term "template strand" as used herein refers to the other strand complementary with the coding strand in the double-stranded DNA of the target gene, i.e. the strand that, as a template, can be transcribed into RNA, and this strand is complementary with the transcribed RNA (A to U, G to C). In the process of transcription, RNA polymerase is bound with the template strand, moves along the 3'→5' direction of the template strand, and catalyzes the synthesis of the RNA along the 5'→3' direction. The template strand of the double-stranded DNA sequence of the target gene promoter described in the present invention refers to a promoter sequence on the same DNA strand as the DNA template strand of the target gene.

The term "promoter" as used herein refers to a nucleic acid sequence, which does not encode a protein, which plays a regulatory role for the transcription of a protein-coding or RNA-coding nucleic acid sequence by associating with them spatially. Generally, a eukaryotic promoter contains 100 to 5,000 base pairs, although this length range is not intended to limit the term "promoter" as used herein. Although the promoter sequence is generally located at the 5' terminus of a protein-coding or RNA-coding sequence, in some cases, the promoter sequence also exists in exon and intron sequences.

The term "transcription start site" as used herein refers to a nucleotide marking the transcription start on the template strand of a gene. The transcription start site can appear on the template strand of the promoter region. A gene can have more than one transcription start site.

The term "sequence identity" or "sequence homology" as used herein means that one oligonucleotide strand (sense or antisense) of a saRNA has at least 75% similarity with a region on the coding strand or template strand of the promoter sequence of a target gene.

The term "overhang" as used herein refers to non-base-paired nucleotides at the terminus (5' or 3') of an oligonucleotide strand, which is formed by one strand extending out of the other strand in a duplex oligonucleotide. A single-stranded region extending out of the 3' terminus and/or 5' terminus of a duplex is referred to as an overhang.

As used herein, the terms "gene activation" or "activating gene expression" can be used interchangeably, and means an increase or upregulation in transcription, translation, expression or activity of a certain nucleic acid as determined by measuring the transcription level, mRNA level, protein level, enzymatic activity, methylation state, chromatin state or configuration, translation level or the activity or state in a cell or biological system of a gene. These activities or states can be determined directly or indirectly. In addition, "gene activation" or "activating gene expression" refers to an increase in activity associated with a nucleic acid sequence, regardless the mechanism of such activation. For example, gene activation occurs at the transcriptional level to increase transcription into RNA and the RNA is translated into a protein, thereby increasing the expression of the protein.

As used herein, the terms "small activating RNA," "saRNA," and "small activating nucleic acid molecule" can be used interchangeably, and refer to a ribonucleic acid molecule that can upregulate target gene expression. The saRNA can be composed of a first ribonucleic acid strand (antisense strand, also referred to as antisense oligonucleotide strand) containing a ribonucleotide sequence having sequence homology with the non-coding nucleic acid sequence (e.g., a promotor and an enhancer) of a target gene and a second ribonucleic acid strand (sense strand, also referred to as sense oligonucleotide strand) containing a nucleotide sequence complementary with the first ribonucleic acid strand, wherein the first ribonucleic acid strand and the second ribonucleic acid strand form a duplex. The saRNA can also be comprised of a synthesized or vector-expressed single-stranded RNA molecule that can form a hairpin structure by two complementary regions within the molecule, wherein the first region contains a nucleic acid sequence having sequence homology with the target sequence of a promoter of a gene, and a nucleic acid sequence contained in the second region is complementary with the first region. The length of the duplex region of the saRNA molecule is typically about 10 to about 50, about 12 to about 48, about 14 to about 46, about 16 to about 44, about 18 to about 42, about 20 to about 40, about 22 to about 38, about 24 to about 36, about 26 to about 34, and about 28 to about 32 base pairs, and typically about 10, about 15, about 20, about 25, about 30, about 35, about 40, about 45, or about 50 base pairs. In addition, the terms "saRNA", "small activating RNA", and "small activating nucleic acid molecule" also contain nucleic acids other than the ribonucleotide, including, but not limited to, modified nucleotides or analogues.

As used herein, the term "hotspot" refers to a promoter region of a gene where targets for functional saRNAs are enriched. In these hotspot regions, at least 60% of the small activating nucleic acid molecules targeting these hotspot regions can induce a 1.5-fold or more change in the mRNA expression of a target gene.

As used herein, the term "p21" refers to the p21^{WAF1/CIP1} gene, also known as the CDKN1A gene. As a cyclin-dependent kinase (CDK) inhibitor, p21 is an important tumor suppressor gene, also known as "target gene" sometimes. Overexpression of p21 or activation of endogenous p21 transcription has been shown to inhibit the growth of cultured tumor cells and tumors in vivo.

As used herein, the term "synthesis" refers to a method for synthesis of an oligonucleotide, including any method allowing RNA synthesis, such as chemical synthesis, in-vitro transcription, and/or vector-based expression. The present invention provides a method for preparing the small activating nucleic acid molecule, which comprises sequence design and sequence synthesis. The synthesis of the sequence of the small activating nucleic acid molecule can adopt a chemical synthesis or can be entrusted to a biotechnology company specialized in nucleic acid synthesis. Generally speaking, the chemical synthesis comprises the following four steps: (1) synthesis of oligomeric ribonucleotides; (2) deprotection; (3) purification and isolation; (4) desalination and annealing. For example, the specific steps for chemically synthesizing the double-stranded RNA molecule of the present invention, such as saRNA, are as follows:

### (1) synthesis of oligomeric ribonucleotides

Synthesis of 1 micromole of RNA was set in an automatic DNA/RNA synthesizer (e.g., Applied Biosystems EXPEDITE8909), and the coupling time of each cycle was also set as 10 to 15 minutes. With a solid phase-bonded 5'-O-p-dimethoxytriphenylmethyl-thymidine substrate as an initiator, one base was bonded to the solid phase substrate in the first cycle, and then, in the nth (19 ≥ n ≥ 2) cycle, one base was bonded to the base bonded in the n-1 cycle. This process was repeated until the synthesis of the whole nucleic acid sequence was completed.

### (2) deprotection

The solid phase substrate bonded with the saRNA was put into a test tube, and 1 ml of a solution of the mixture of ethanol and ammonium hydroxide (volume ratio: 1:3) was added into the test tube. The test tube was then sealed and placed in an incubator, and the mixture was incubated at 25-70°C for 2 to 30 hours. The solution containing the solid phase substrate bonded with the saRNA was filtered, and the filtrate was collected. The solid phase substrate was rinsed with double distilled water twice (1 ml each time), and the filtrate was collected. The eluents were combined and collected, and dried under vacuum for 1 to 12 hours. Then, 1 ml of a solution of tetrabutylammonium fluoride in tetrahydrofuran (1 M) was added. After 4 to 12 hours of standing at room temperature, 2 ml of n-butanol was then added. Precipitate was collected to obtain a single-stranded crude product of saRNA by highspeed centrifugation.

### (3) purification and isolation

The obtained crude product of saRNA was dissolved in 2 ml of aqueous ammonium acetate solution with a concentration of 1 mol/ml, and the solution was separated by a reversed-phase C18 column of high pressure liquid chromatography to obtain a purified single-stranded product of saRNA.

### (4) desalination and annealing

Salts were removed by gel filtration (size exclusion chromatography). A single sense oligomeric ribonucleic acid strand and a single antisense oligomeric ribonucleic acid strand were mixed into 1 to 2 ml of buffer (10 mM Tris, pH = 7.5-8.0, 50 mM NaCl) at a molar ratio of 1:1. The solution was heated to 95 °C, and was then slowly cooled to room temperature to obtain a solution containing saRNA.

### Materials and methods

### Cell culture and transfection

Cell lines RT4, KU-7, T24, J82, TCCSUP, and HT-1197 were cultured in the modified McCoy's 5A medium (Gibco); cell lines 5637, PC3, and Bel-7402 were cultured in RPMI1640 medium (Gibco); and the cell line UM-UC-3 was cultured in basal medium (Gibco). All the media contained 10% bovine calf serum (Sigma-Aldrich) and 1% penicillin/streptomycin (Gibco). The cells were cultured at 5% CO₂ and 37°C. Double-stranded RNA molecules designed in the experiment were transfected into cells using RNAiMax (Invitrogen, Carlsbad, CA) according to the instructions provided by the manufacturer at the concentration of 10 nM (unless otherwise specified).

### RNA isolation and reverse transcription-polymerase chain reaction

Cells were seeded into a 6-well plate with 2-3 × 10⁵ cells/well and were reverse transfected with oligonucleotide duplexes. At the end of the transfection, total cellular RNA was isolated using an RNeasy Plus Mini kit (Qiagen; Hilden, Germany) according to its manual. The isolated RNA (1 µg) was reverse transcribed into cDNA by using a PrimeScript RT kit containing gDNA Eraser (Takara, Shiga, Japan). The resulted cDNA was amplified in an ABI 7500 Fast Real-time PCR System (Applied Biosystems; Foster City, CA) using SYBR Premix Ex Taq II (Takara, Shiga, Japan) reagents and target gene specific primers. The reaction conditions were: 95°C for 3 seconds, 60°C for 30 seconds, and 40 cycles. Amplification of GAPDH served as an internal control. All primer sequences are listed in Table 1.

**Table 1: Primer sequences for RT-qPCR assay**

| **Primer Title** | **Sequence No.** | **Sequence (5'-3')** |
|---|---|---|
| GAPDH F | SEQ ID NO 1 | ATCACCATCTTCCAGGAGCGA |
| GAPDH R | SEQ ID NO 2 | TTCTCCATGGTGGTGAAGACG |
| CDKN1A F | SEQ ID NO 3 | GGAAGACCATGTGGACCTGT |
| CDKN1A R | SEQ ID NO 4 | GGATTAGGGCTTCCTCTTGG |

### Assay of cell proliferation

Cells were plated into a 96-well plate with 2-4 × 10³ cells/well, cultured overnight, and transfected with oligonucleotide duplexes. Three days after transfection, the CCK8 reagent (Dojindo; Rockville, MD) was used to assay the cell proliferation according to its manual. Briefly, 10 µL of CCK8 reagent was added into each well on the plate which was then incubated at 37°C for 1 hour. Absorbance for each well on the plate was measured at 450 nm by a microplate reader.

### QuantiGene 2.0 assay

Cells were plated into a 96-well plate and transfected with oligonucleotide duplexes. 72 hours after transfection, the mRNA levels of target genes were quantitatively assayed with a QuantiGene 2.0 kit (AffyMetrix; Santa Clara, CA). QuantiGene 2.0 assay is based on hybridization technology, wherein mRNA levels were directly quantified with gene-specific probes. The experimental procedure is briefly described as follows: a lysis solution was added to lyse the transfected cells, and the cell lysates were added into a capture well plate coated with probe for CDKN1A (p21) and HPRT1 which serves as a housekeeping gene for hybridizing overnight at 55°C. In order to enhance the hybridization signal, hybridizations with 2.0 PreAMP Probe, 2.0 AMP Probe, and 2.0 Label Probe were conducted sequentially in 100 µL of a corresponding buffer solution (provided by Quantigene 2.0 kit). All the hybridizations were conducted with shaking at 50-55°C for 1 hour. After the last wash step, 2.0 Substrate was added to the solution and incubated at room temperature for 5 minutes. Optical signals were detected with an Infinite 200 PRO plate reader (Tecan, Switzerland).

### Statistical analysis

Results were represented as mean ± standard deviation. One-way analysis of variance was carried out with GraphPad Prism software (GraphPad Software), and then a Tukey's t test was conducted for pairwise comparisons. A statistical significance was set as * p < 0.05, ** p < 0.01, and *** p < 0.001.

### Examples

The present invention is further illustrated by the following examples. These examples are provided merely for illustration purposes and shall not be interpreted to limit the scope or content of the present invention in any way.

### Example 1: Screening of functional saRNAs targeting the promoter region of p21

The 1 kb promoter sequence (Fig. 1) (SEQ ID NO:469) of p21 was retrieved from the UCSC Genome database to screen for functional saRNAs capable of activating p21 gene expression. A total of 982 target sequences were obtained by selecting a target with a size of 19 bp starting from the -1 kb position upstream of TSS and moving toward the TSS one base pair (bp) at a time. The target sequences were filtered to remove those that have a GC content higher than 65% or lower than 35% and those that contain 5 or more consecutive nucleotides. After filtration of the target sequences, 439 target sequences remained and were used as candidates for screening. Corresponding double-stranded RNA molecules were chemically synthesized based on these candidate sequences. Each of the sense strand and antisense strand in the double-stranded RNA molecule used in the experiment had 21 nucleotides in length. The 19 nucleotides in the 5' region of the first ribonucleic acid strand (sense strand) of the double-stranded RNA molecule (e.g., double-stranded saRNA) had 100% homology with the target sequence of the promoter, and the 3' terminus of the first ribonucleic acid strand contained a dTdT overhang. The 19 nucleotides in the 5' region of the second ribonucleic acid strand were fully complementary with the 19 nucleotides in the 5' region sequence of the first ribonucleic acid strand, and the 3' terminus of the second ribonucleic acid strand contained a dTdT overhang.

The aforementioned double-stranded RNA molecules were transfected into PC3 prostate cancer cells at a final concentration of 10 nM, and 72 hours after transfection, the p21 mRNA level was detected with the QuantiGene 2.0 kit. The fold change of p21 mRNA level induced by each double-stranded RNA molecule relative to the blank control was calculated and plotted in Fig. 2. In this study, the fold changes of p21 mRNA level induced by all double-stranded RNA molecules ranged from 0.66 (suppression) to 8.12 (induction) (Fig. 2B). There were 361 (82.2%) double-stranded RNA molecules inducing a 1.01-fold to 8.12-fold change of p21 expression, 74 (16.9%) double-stranded RNA molecules exhibiting a suppressing effect (0.99-fold to 0.66-fold change), and 4 (0.9%) double-stranded RNA molecules having no influence on p21 mRNA level (1.0-fold change).

Among the 439 screened double-stranded RNA molecules, 132 double-stranded RNA molecules (30.1%) could induce at least a 2-fold change in p21 mRNA level, and 229 (52.4%) double-stranded RNA molecules could induce at least a 1.5-fold change in p21 mRNA level. These double-stranded RNA molecules that upregulated p21 mRNA expression by more than 10% were functional saRNAs. The targets for these functional saRNAs were scattered in the entire p21 promoter region. However, 8 discrete regions showed enrichment for saRNA targets and these regions are considered as saRNA "hotspots." The hotspot is defined as a region containing at least 10 corresponding saRNAs, wherein at least 60% of them could induce a 1.5-fold or more change in p21 mRNA expression (Fig. 2A and Fig. 3). The target sequences for hotspots 1-8 and corresponding saRNA sequences are listed in **Table 2** and **Table 3** respectively.

**Table 2: Target sequences of hotspot regions of p21 promoter**

| **Hotspot** | **Sequence No.** | **DNA Sequence (5'-3')** | **Location (relative to TSS)** | **Length (bp)** |
|---|---|---|---|---|
| Hotspot 1 | SEQ ID NO: 5 | ggctatgtggggagtattcaggagacagacaactcactcgt caaatcctccccttcctggccaacaaagctgctgcaaccac agggatttct | -893∼-801 | 92 |
| Hotspot 2 | SEQ ID NO: 6 | ggtagtctctccaattccctccttcccggaagcatgtgacaat caacaactttgtatacttaagttcagtggacctcaatttcctc | -717∼-632 | 86 |
| Hotspot 3 | SEQ ID NO: 7 | ctttgttggggtgtctaggtgctccaggtgcttct | -585∼-551 | 35 |
| Hotspot 4 | SEQ ID NO: 8 | ttctgggagaggtgacctagtgagggatcagtgggaataga ggtgatattg | -554∼-504 | 51 |
| Hotspot 5 | SEQ ID NO: 9 | aggtgatattgtggggcttttctggaaatt | -514∼-485 | 30 |
| Hotspot 6 | SEQ ID NO: 10 | attaatgtcatcctcctgatcttttcagctgcattggg | -442∼-405 | 38 |
| Hotspot 7 | SEQ ID NO: 11 | tctaacagtgctgtgtcctcctggagagtgccaactcatt | -352∼-313 | 40 |
| Hotspot 8 | SEQ ID NO: 12 | gtgccaactcattctccaagtaaaaaaagccagatttgtggct cacttcgtggggaaatgtgtcca | -325∼-260 | 66 |

**Table 3: Screened double-stranded RNA molecules located in hotspot regions of p21 promoter**

| **Title** | **Sequence No.** | **Sequence (5'-3')** | **Length** |
|---|---|---|---|
| **Hotspot 8** | | | |
| RAG-278 | SEQ ID NO: 13 | UCGUGGGGAAAUGUGUCCA[dT][dT] | 21 nt |
| | SEQ ID NO: 14 | UGGACACAUUUCCCCACGA[dT] [dT] | 21 nt |
| RAG-279 | SEQ ID NO: 15 | UUCGUGGGGAAAUGUGUCC[dT][dT] | 21 nt |
| | SEQ ID NO: 16 | GGACACAUUUCCCCACGAA[dT] [dT] | 21 nt |
| RAG-280 | SEQ ID NO: 17 | CUUCGUGGGGAAAUGUGUC[dT][dT] | 21 nt |
| | SEQ ID NO: 18 | GACACAUUUCCCCACGAAG[dT] [dT] | 21 nt |
| RAG-281 | SEQ ID NO: 19 | ACUUCGUGGGGAAAUGUGU[dT][dT] | 21 nt |
| | SEQ ID NO: 20 | ACACAUUUCCCCACGAAGU[dT][dT] | 21 nt |
| RAG-282 | SEQ ID NO: 21 | CACUUCGUGGGGAAAUGUG[dT][dT] | 21 nt |
| | SEQ ID NO: 22 | CACAUUUCCCCACGAAGUG[dT] [dT] | 21 nt |
| RAG-283 | SEQ ID NO: 23 | UCACUUCGUGGGGAAAUGU[dT][dT] | 21 nt |
| | SEQ ID NO: 24 | ACAUUUCCCCACGAAGUGA[dT] [dT] | 21 nt |
| RAG-284 | SEQ ID NO: 25 | CUCACUUCGUGGGGAAAUG[dT][dT] | 21 nt |
| | SEQ ID NO: 26 | CAUUUCCCCACGAAGUGAG[dT] [dT] | 21 nt |
| RAG-285 | SEQ ID NO: 27 | GCUCACUUCGUGGGGAAAU[dT][dT] | 21 nt |
| | SEQ ID NO: 28 | AUUUCCCCACGAAGUGAGC[dT] [dT] | 21 nt |
| RAG-286 | SEQ ID NO: 29 | GGCUCACUUCGUGGGGAAA[dT] [dT] | 21 nt |
| | SEQ ID NO: 30 | UUUCCCCACGAAGUGAGCC[dT] [dT] | 21 nt |
| RAG-287 | SEQ ID NO: 31 | UGGCUCACUUCGUGGGGAA[dT] [dT] | 21 nt |
| | SEQ ID NO: 32 | UUCCCCACGAAGUGAGCCA[dT] [dT] | 21 nt |
| RAG-288 | SEQ ID NO: 33 | GUGGCUCACUUCGUGGGGA[dT] [dT] | 21 nt |
| | SEQ ID NO: 34 | UCCCCACGAAGUGAGCCAC[dT] [dT] | 21 nt |
| RAG-289 | SEQ ID NO: 35 | UGUGGCUCACUUCGUGGGG[dT] [dT] | 21 nt |
| | SEQ ID NO: 36 | CCCCACGAAGUGAGCCACA[dT] [dT] | 21 nt |
| RAG-291 | SEQ ID NO: 37 | UUUGUGGCUCACUUCGUGG[dT] [dT] | 21 nt |
| | SEQ ID NO: 38 | CCACGAAGUGAGCCACAAA[dT] [dT] | 21 nt |
| RAG-292 | SEQ ID NO: 39 | AUUUGUGGCUCACUUCGUG[dT] [dT] | 21 nt |
| | SEQ ID NO: 40 | CACGAAGUGAGCCACAAAU[dT][dT] | 21 nt |
| RAG-293 | SEQ ID NO: 41 | GAUUUGUGGCUCACUUCGU[dT][dT] | 21 nt |
| | SEQ ID NO: 42 | ACGAAGUGAGCCACAAAUC[dT][dT] | 21 nt |
| RAG-294 | SEQ ID NO: 43 | AGAUUUGUGGCUCACUUCG[dT][dT] | 21 nt |
| | SEQ ID NO: 44 | CGAAGUGAGCCACAAAUCU[dT][dT] | 21 nt |
| RAG-295 | SEQ ID NO: 45 | CAGAUUUGUGGCUCACUUC[dT] [dT] | 21 nt |
| | SEQ ID NO: 46 | GAAGUGAGCCACAAAUCUGdT][dT] | 21 nt |
| RAG-296 | SEQ ID NO: 47 | CCAGAUUUGUGGCUCACUU[dT][dT] | 21 nt |
| | SEQ ID NO: 48 | AAGUGAGCCACAAAUCUGG[dT][dT] | 21 nt |
| RAG-297 | SEQ ID NO: 49 | GCCAGAUUUGUGGCUCACU[dT][dT] | 21 nt |
| | SEQ ID NO: 50 | AGUGAGCCACAAAUCUGGC[dT][dT] | 21 nt |
| RAG-298 | SEQ ID NO: 51 | AGCCAGAUUUGUGGCUCAC[dT][dT] | 21 nt |
| | SEQ ID NO: 52 | GUGAGCCACAAAUCUGGCU[dT][dT] | 21 nt |
| RAG-299 | SEQ ID NO: 53 | AAGCCAGAUUUGUGGCUCA[dT][dT] | 21 nt |
| | SEQ ID NO: 54 | UGAGCCACAAAUCUGGCUU[dT][dT] | 21 nt |
| RAG-300 | SEQ ID NO: 55 | AAAGCCAGAUUUGUGGCUC[dT][dT] | 21 nt |
| | SEQ ID NO: 56 | GAGCCACAAAUCUGGCUUU[dT][dT] | 21 nt |
| RAG-301 | SEQ ID NO: 57 | AAAAGCCAGAUUUGUGGCU[dT][dT] | 21 nt |
| | SEQ ID NO: 58 | AGCCACAAAUCUGGCUUUU[dT][dT] | 21 nt |
| RAG-321 | SEQ ID NO: 59 | CAACUCAUUCUCCAAGUAA[dT] [dT] | 21 nt |
| | SEQ ID NO: 60 | UUACUUGGAGAAUGAGUUG[dT][dT] | 21 nt |
| RAG-322 | SEQ ID NO: 61 | CCAACUCAUUCUCCAAGUA[dT][dT] | 21 nt |
| | SEQ ID NO: 62 | UACUUGGAGAAUGAGUUGG[dT][dT] | 21 nt |
| RAG-323 | SEQ ID NO: 63 | GCCAACUCAUUCUCCAAGU[dT][dT] | 21 nt |
| | SEQ ID NO: 64 | ACUUGGAGAAUGAGUUGGC[dT][dT] | 21 nt |
| RAG-324 | SEQ ID NO: 65 | UGCCAACUCAUUCUCCAAG[dT][dT] | 21 nt |
| | SEQ ID NO: 66 | CUUGGAGAAUGAGUUGGCA[dT][dT] | 21 nt |
| RAG-325 | SEQ ID NO: 67 | GUGCCAACUCAUUCUCCAA[dT][dT] | 21 nt |
| | SEQ ID NO: 68 | UUGGAGAAUGAGUUGGCAC[dT][dT] | 21 nt |

| **Hotspot 7** | | | |
|---|---|---|---|
| RAG-331 | SEQ ID NO: 69 | UGGAGAGUGCCAACUCAUU[dT][dT] | 21 nt |
| | SEQ ID NO: 70 | AAUGAGUUGGCACUCUCCA[dT][dT] | 21 nt |
| RAG-332 | SEQ ID NO: 71 | CUGGAGAGUGCCAACUCAU[dT][dT] | 21 nt |
| | SEQ ID NO: 72 | AUGAGUUGGCACUCUCCAG[dT] [dT] | 21 nt |
| RAG-333 | SEQ ID NO: 73 | CCUGGAGAGUGCCAACUCA[dT] [dT] | 21 nt |
| | SEQ ID NO: 74 | UGAGUUGGCACUCUCCAGG[dT] [dT] | 21 nt |
| RAG-334 | SEQ ID NO: 75 | UCCUGGAGAGUGCCAACUC[dT] [dT] | 21 nt |
| | SEQ ID NO: 76 | GAGUUGGCACUCUCCAGGA[dT] [dT] | 21 nt |
| RAG-335 | SEQ ID NO: 77 | CUCCUGGAGAGUGCCAACU[dT][dT] | 21 nt |
| | SEQ ID NO: 78 | AGUUGGCACUCUCCAGGAG[dT][dT] | 21 nt |
| RAG-336 | SEQ ID NO: 79 | CCUCCUGGAGAGUGCCAAC[dT] [dT] | 21 nt |
| | SEQ ID NO: 80 | GUUGGCACUCUCCAGGAGG[dT] [dT] | 21 nt |
| RAG-337 | SEQ ID NO: 81 | UCCUCCUGGAGAGUGCCAA[dT] [dT] | 21 nt |
| | SEQ ID NO: 82 | UUGGCACUCUCCAGGAGGA[dT] [dT] | 21 nt |
| RAG-338 | SEQ ID NO: 83 | GUCCUCCUGGAGAGUGCCA[dT][dT] | 21 nt |
| | SEQ ID NO: 84 | UGGCACUCUCCAGGAGGAC[dT][dT] | 21 nt |
| RAG-341 | SEQ ID NO: 85 | UGUGUCCUCCUGGAGAGUG[dT] [dT] | 21 nt |
| | SEQ ID NO: 86 | CACUCUCCAGGAGGACACA[dT][dT] | 21 nt |
| RAG-342 | SEQ ID NO: 87 | CUGUGUCCUCCUGGAGAGU[dT][dT] | 21 nt |
| | SEQ ID NO: 88 | ACUCUCCAGGAGGACACAG[dT][dT] | 21 nt |
| RAG-343 | SEQ ID NO: 89 | GCUGUGUCCUCCUGGAGAG[dT] [dT] | 21 nt |
| | SEQ ID NO: 90 | CUCUCCAGGAGGACACAGC[dT][dT] | 21 nt |
| RAG-344 | SEQ ID NO: 91 | UGCUGUGUCCUCCUGGAGA[dT][dT] | 21 nt |
| | SEQ ID NO: 92 | UCUCCAGGAGGACACAGCA[dT] [dT] | 21 nt |
| RAG-345 | SEQ ID NO: 93 | GUGCUGUGUCCUCCUGGAG[dT][dT] | 21 nt |
| | SEQ ID NO: 94 | CUCCAGGAGGACACAGCAC[dT][dT] | 21 nt |
| RAG-346 | SEQ ID NO: 95 | AGUGCUGUGUCCUCCUGGA[dT][dT] | 21 nt |
| | SEQ ID NO: 96 | UCCAGGAGGACACAGCACU[dT][dT] | 21 nt |
| RAG-348 | SEQ ID NO: 97 | ACAGUGCUGUGUCCUCCUG[dT][dT] | 21 nt |
| | SEQ ID NO: 98 | CAGGAGGACACAGCACUGU[dT][dT] | 21 nt |
| RAG-349 | SEQ ID NO: 99 | AACAGUGCUGUGUCCUCCU[dT][dT] | 21 nt |
| | SEQ ID NO: 100 | AGGAGGACACAGCACUGUU[dT][dT] | 21 nt |
| RAG-350 | SEQ ID NO: 101 | UAACAGUGCUGUGUCCUCC[dT][dT] | 21 nt |
| | SEQ ID NO: 102 | GGAGGACACAGCACUGUUA[dT] [dT] | 21 nt |
| RAG-3 51 | SEQ ID NO: 103 | CUAACAGUGCUGUGUCCUC[dT][dT] | 21 nt |
| | SEQ ID NO: 104 | GAGGACACAGCACUGUUAG[dT] [dT] | 21 nt |
| RAG-352 | SEQ ID NO: 105 | UCUAACAGUGCUGUGUCCU[dT][dT] | 21 nt |
| | SEQ ID NO: 106 | AGGACACAGCACUGUUAGA[dT] [dT] | 21 nt |

| **Hotspot 6** | | | |
|---|---|---|---|
| RAG-423 | SEQ ID NO: 107 | UCUUUUCAGCUGCAUUGGG[dT][dT] | 21 nt |
| | SEQ ID NO: 108 | CCCAAUGCAGCUGAAAAGA[dT][dT] | 21 nt |
| RAG-424 | SEQ ID NO: 109 | AUCUUUUCAGCUGCAUUGG[dT][dT] | 21 nt |
| | SEQ ID NO: 110 | CCAAUGCAGCUGAAAAGAU[dT][dT] | 21 nt |
| RAG-425 | SEQ ID NO: 111 | GAUCUUUUCAGCUGCAUUG[dT][dT] | 21 nt |
| | SEQ ID NO: 112 | CAAUGCAGCUGAAAAGAUC[dT][dT] | 21 nt |
| RAG-426 | SEQ ID NO: 113 | UGAUCUUUUCAGCUGCAUU[dT][dT] | 21 nt |
| | SEQ ID NO: 114 | AAUGCAGCUGAAAAGAUCA[dT][dT] | 21 nt |
| RAG-427 | SEQ ID NO: 115 | CUGAUCUUUUCAGCUGCAU[dT][dT] | 21 nt |
| | SEQ ID NO: 116 | AUGCAGCUGAAAAGAUCAG[dT][dT] | 21 nt |
| RAG-428 | SEQ ID NO: 117 | CCUGAUCUUUUCAGCUGCA[dT][dT] | 21 nt |
| | SEQ ID NO: 118 | UGCAGCUGAAAAGAUCAGG[dT][dT] | 21 nt |
| RAG-429 | SEQ ID NO: 119 | UCCUGAUCUUUUCAGCUGC[dT][dT] | 21 nt |
| | SEQ ID NO: 120 | GCAGCUGAAAAGAUCAGGA[dT][dT] | 21 nt |
| RAG-430 | SEQ ID NO: 121 | CUCCUGAUCUUUUCAGCUG[dT] [dT] | 21 nt |
| | SEQ ID NO: 122 | CAGCUGAAAAGAUCAGGAG[dT][dT] | 21 nt |
| RAG-431 | SEQ ID NO: 123 | CCUCCUGAUCUUUUCAGCU[dT][dT] | 21 nt |
| | SEQ ID NO: 124 | AGCUGAAAAGAUCAGGAGG[dT][dT] | 21 nt |
| RAG-432 | SEQ ID NO: 125 | UCCUCCUGAUCUUUUCAGC[dT][dT] | 21 nt |
| | SEQ ID NO: 126 | GCUGAAAAGAUCAGGAGGA[dT][dT] | 21 nt |
| RAG-433 | SEQ ID NO: 127 | AUCCUCCUGAUCUUUUCAG[dT] [dT] | 21 nt |
| | SEQ ID NO: 128 | CUGAAAAGAUCAGGAGGAU[dT][dT] | 21 nt |
| RAG-434 | SEQ ID NO: 129 | CAUCCUCCUGAUCUUUUCA[dT][dT] | 21 nt |
| | SEQ ID NO: 130 | UGAAAAGAUCAGGAGGAUG[dT][dT] | 21 nt |
| RAG-435 | SEQ ID NO: 131 | UCAUCCUCCUGAUCUUUUC[dT][dT] | 21 nt |
| | SEQ ID NO: 132 | GAAAAGAUCAGGAGGAUGA[dT][dT] | 21 nt |
| RAG-436 | SEQ ID NO: 133 | GUCAUCCUCCUGAUCUUUU[dT][dT] | 21 nt |
| | SEQ ID NO: 134 | AAAAGAUCAGGAGGAUGAC[dT][dT] | 21 nt |
| RAG-437 | SEQ ID NO: 135 | UGUCAUCCUCCUGAUCUUU[dT][dT] | 21 nt |
| | SEQ ID NO: 136 | AAAGAUCAGGAGGAUGACA[dT][dT] | 21 nt |
| RAG-438 | SEQ ID NO: 137 | AUGUCAUCCUCCUGAUCUU[dT][dT] | 21 nt |
| | SEQ ID NO: 138 | AAGAUCAGGAGGAUGACAU[dT][dT] | 21 nt |
| RAG-439 | SEQ ID NO: 139 | AAUGUCAUCCUCCUGAUCU[dT][dT] | 21 nt |
| | SEQ ID NO: 140 | AGAUCAGGAGGAUGACAUU[dT][dT] | 21 nt |
| RAG-440 | SEQ ID NO: 141 | UAAUGUCAUCCUCCUGAUC[dT] [dT] | 21 nt |
| | SEQ ID NO: 142 | GAUCAGGAGGAUGACAUUA[dT] [dT] | 21 nt |
| RAG-441 | SEQ ID NO: 143 | UUAAUGUCAUCCUCCUGAU[dT] [dT] | 21 nt |
| | SEQ ID NO: 144 | AUCAGGAGGAUGACAUUAA[dT] [dT] | 21 nt |
| RAG-442 | SEQ ID NO: 145 | AUUAAUGUCAUCCUCCUGA[dT] [dT] | 21 nt |
| | SEQ ID NO: 146 | UCAGGAGGAUGACAUUAAU[dT][dT] | 21 nt |

| **Hotspot 5** | | | |
|---|---|---|---|
| RAG-503 | SEQ ID NO: 147 | UGGGGCUUUUCUGGAAAUU[dT][dT] | 21 nt |
| | SEQ ID NO: 148 | AAUUUCCAGAAAAGCCCCA[dT] [dT] | 21 nt |
| RAG-504 | SEQ ID NO: 149 | GUGGGGCUUUUCUGGAAAU[dT][dT] | 21 nt |
| | SEQ ID NO: 150 | AUUUCCAGAAAAGCCCCAC[dT][dT] | 21 nt |
| RAG-505 | SEQ ID NO: 151 | UGUGGGGCUUUUCUGGAAA[dT][dT] | 21 nt |
| | SEQ ID NO: 152 | UUUCCAGAAAAGCCCCACA[dT] [dT] | 21 nt |
| RAG-506 | SEQ ID NO: 153 | UUGUGGGGCUUUUCUGGAA[dT][dT] | 21 nt |
| | SEQ ID NO: 154 | UUCCAGAAAAGCCCCACAA[dT] [dT] | 21 nt |
| RAG-507 | SEQ ID NO: 155 | AUUGUGGGGCUUUUCUGGA[dT][dT] | 21 nt |
| | SEQ ID NO: 156 | UCCAGAAAAGCCCCACAAU[dT][dT] | 21 nt |
| RAG-508 | SEQ ID NO: 157 | UAUUGUGGGGCUUUUCUGG[dT][dT] | 21 nt |
| | SEQ ID NO: 158 | CCAGAAAAGCCCCACAAUA[dT] [dT] | 21 nt |
| RAG-509 | SEQ ID NO: 159 | AUAUUGUGGGGCUUUUCUG[dT][dT] | 21 nt |
| | SEQ ID NO: 160 | CAGAAAAGCCCCACAAUAU[dT][dT] | 21 nt |
| RAG-510 | SEQ ID NO: 161 | GAUAUUGUGGGGCUUUUCU[dT][dT] | 21 nt |
| | SEQ ID NO: 162 | AGAAAAGCCCCACAAUAUC[dT] [dT] | 21 nt |
| RAG-511 | SEQ ID NO: 163 | UGAUAUUGUGGGGCUUUUC[dT] [dT] | 21 nt |
| | SEQ ID NO: 164 | GAAAAGCCCCACAAUAUCA[dT] [dT] | 21 nt |
| RAG-512 | SEQ ID NO: 165 | GUGAUAUUGUGGGGCUUUU[dT][dT] | 21 nt |
| | SEQ ID NO: 166 | AAAAGCCCCACAAU AUCAC[dT] [dT] | 21 nt |
| RAG-513 | SEQ ID NO: 167 | GGUGAUAUUGUGGGGCUUU[dT][dT] | 21 nt |
| | SEQ ID NO: 168 | AAAGCCCCACAAUAUCACC[dT] [dT] | 21 nt |
| RAG-514 | SEQ ID NO: 169 | AGGUGAUAUUGUGGGGCUU[dT][dT] | 21 nt |
| | SEQ ID NO: 170 | AAGCCCCACAAUAUCACCU[dT][dT] | 21 nt |
| **Hotspot 4** | | | |
| RAG-522 | SEQ ID NO: 171 | GGGAAUAGAGGUGAUAUUG[dT][dT] | 21 nt |
| | SEQ ID NO: 172 | CAAUAUCACCUCUAUUCCC[dT][dT] | 21 nt |
| RAG-523 | SEQ ID NO: 173 | UGGGAAUAGAGGUGAUAUU[dT][dT] | 21 nt |
| | SEQ ID NO: 174 | AAUAUCACCUCUAUUCCCA[dT] [dT] | 21 nt |
| RAG-524 | SEQ ID NO: 175 | GUGGGAAUAGAGGUGAU AU [dT] [dT] | 21 nt |
| | SEQ ID NO: 176 | AUAUCACCUCUAUUCCCAC[dT] [dT] | 21 nt |
| RAG-525 | SEQ ID NO: 177 | AGUGGGAAUAGAGGUGAUA[dT][dT] | 21 nt |
| | SEQ ID NO: 178 | UAUCACCUCUAUUCCCACU[dT][dT] | 21 nt |
| RAG-526 | SEQ ID NO: 179 | CAGUGGGAAUAGAGGUGAU[dT][dT] | 21 nt |
| | SEQ ID NO: 180 | AUCACCUCUAUUCCCACUG[dT] [dT] | 21 nt |
| RAG-527 | SEQ ID NO: 181 | UCAGUGGGAAUAGAGGUGA[dT][dT] | 21 nt |
| | SEQ ID NO: 182 | UCACCUCUAUUCCCACUGA[dT] [dT] | 21 nt |
| RAG-528 | SEQ ID NO: 183 | AUCAGUGGGAAUAGAGGUG[dT][dT] | 21 nt |
| | SEQ ID NO: 184 | CACCUCUAUUCCCACUGAU[dT][dT] | 21 nt |
| RAG-529 | SEQ ID NO: 185 | GAUCAGUGGGAAUAGAGGU[dT][dT] | 21 nt |
| | SEQ ID NO: 186 | ACCUCUAUUCCCACUGAUC[dT] [dT] | 21 nt |
| RAG-530 | SEQ ID NO: 187 | GGAUCAGUGGGAAUAGAGG[dT] [dT] | 21 nt |
| | SEQ ID NO: 188 | CCUCUAUUCCCACUGAUCC[dT][dT] | 21 nt |
| RAG-531 | SEQ ID NO: 189 | GGGAUCAGUGGGAAUAGAG[dT] [dT] | 21 nt |
| | SEQ ID NO: 190 | CUCUAUUCCCACUGAUCCC[dT][dT] | 21 nt |
| RAG-532 | SEQ ID NO: 191 | AGGGAUCAGUGGGAAUAGA[dT] [dT] | 21 nt |
| | SEQ ID NO: 192 | UCUAUUCCCACUGAUCCCU[dT][dT] | 21 nt |
| RAG-533 | SEQ ID NO: 193 | GAGGGAUCAGUGGGAAUAG[dT] [dT] | 21 nt |
| | SEQ ID NO: 194 | CUAUUCCCACUGAUCCCUC[dT] [dT] | 21 nt |
| RAG-534 | SEQ ID NO: 195 | UGAGGGAUCAGUGGGAAUA[dT][dT] | 21 nt |
| | SEQ ID NO: 196 | UAUUCCCACUGAUCCCUCA[dT] [dT] | 21 nt |
| RAG-535 | SEQ ID NO: 197 | GUGAGGGAUCAGUGGGAAU[dT][dT] | 21 nt |
| | SEQ ID NO: 198 | AUUCCCACUGAUCCCUCAC[dT][dT] | 21 nt |
| RAG-536 | SEQ ID NO: 199 | AGUGAGGGAUCAGUGGGAA[dT] [dT] | 21 nt |
| | SEQ ID NO: 200 | UUCCCACUGAUCCCUCACU[dT][dT] | 21 nt |
| RAG-537 | SEQ ID NO: 201 | UAGUGAGGGAUCAGUGGGA[dT] [dT] | 21 nt |
| | SEQ ID NO: 202 | UCCCACUGAUCCCUCACUA[dT][dT] | 21 nt |
| RAG-538 | SEQ ID NO: 203 | CUAGUGAGGGAUCAGUGGG[dT][dT] | 21 nt |
| | SEQ ID NO: 204 | CCCACUGAUCCCUCACUAG[dT][dT] | 21 nt |
| RAG-540 | SEQ ID NO: 205 | ACCUAGUGAGGGAUCAGUG[dT][dT] | 21 nt |
| | SEQ ID NO: 206 | CACUGAUCCCUCACUAGGU[dT][dT] | 21 nt |
| RAG-541 | SEQ ID NO: 207 | GACCUAGUGAGGGAUCAGU[dT][dT] | 21 nt |
| | SEQ ID NO: 208 | ACUGAUCCCUCACUAGGUC[dT][dT] | 21 nt |
| RAG-542 | SEQ ID NO: 209 | UGACCUAGUGAGGGAUCAG[dT][dT] | 21 nt |
| | SEQ ID NO: 210 | CUGAUCCCUCACUAGGUCA[dT] [dT] | 21 nt |
| RAG-543 | SEQ ID NO: 211 | GUGACCUAGUGAGGGAUCA[dT][dT] | 21 nt |
| | SEQ ID NO: 212 | UGAUCCCUCACUAGGUCAC[dT] [dT] | 21 nt |
| RAG-544 | SEQ ID NO: 213 | GGUGACCUAGUGAGGGAUC[dT][dT] | 21 nt |
| | SEQ ID NO: 214 | GAUCCCUCACUAGGUCACC[dT] [dT] | 21 nt |
| RAG-545 | SEQ ID NO: 215 | AGGUGACCUAGUGAGGGAU[dT][dT] | 21 nt |
| | SEQ ID NO: 216 | AUCCCUCACUAGGUCACCU[dT][dT] | 21 nt |
| RAG-546 | SEQ ID NO: 217 | GAGGUGACCUAGUGAGGGA[dT] [dT] | 21 nt |
| | SEQ ID NO: 218 | UCCCUCACUAGGUCACCUC[dT][dT] | 21 nt |
| RAG-549 | SEQ ID NO: 219 | GGAGAGGUGACCUAGUGAG[dT] [dT] | 21 nt |
| | SEQ ID NO: 220 | CUCACUAGGUCACCUCUCC[dT] [dT] | 21 nt |
| RAG-550 | SEQ ID NO: 221 | GGGAGAGGUGACCUAGUGA[dT] [dT] | 21 nt |
| | SEQ ID NO: 222 | UCACUAGGUCACCUCUCCC[dT] [dT] | 21 nt |
| RAG-551 | SEQ ID NO: 223 | UGGGAGAGGUGACCUAGUG[dT] [dT] | 21 nt |
| | SEQ ID NO: 224 | CACUAGGUCACCUCUCCCA[dT] [dT] | 21 nt |
| RAG-552 | SEQ ID NO: 225 | CUGGGAGAGGUGACCUAGU[dT][dT] | 21 nt |
| | SEQ ID NO: 226 | ACUAGGUCACCUCUCCCAG[dT][dT] | 21 nt |
| RAG-553 | SEQ ID NO: 227 | UCUGGGAGAGGUGACCUAG[dT] [dT] | 21 nt |
| | SEQ ID NO: 228 | CUAGGUCACCUCUCCCAGA[dT][dT] | 21 nt |
| RAG-554 | SEQ ID NO: 229 | UUCUGGGAGAGGUGACCUA[dT] [dT] | 21 nt |
| | SEQ ID NO: 230 | UAGGUCACCUCUCCCAGAA[dT] [dT] | 21 nt |
| **Hotspot 3** | | | |
| RAG-569 | SEQ ID NO: 231 | AGGUGCUCCAGGUGCUUCU[dT][dT] | 21 nt |
| | SEQ ID NO: 232 | AGAAGCACCUGGAGCACCU[dT][dT] | 21 nt |
| RAG-570 | SEQ ID NO: 233 | UAGGUGCUCCAGGUGCUUC[dT][dT] | 21 nt |
| | SEQ ID NO: 234 | GAAGCACCUGGAGCACCUA[dT] [dT] | 21 nt |
| RAG-574 | SEQ ID NO: 235 | UGUCUAGGUGCUCCAGGUG[dT] [dT] | 21 nt |
| | SEQ ID NO: 236 | CACCUGGAGCACCUAGACA[dT] [dT] | 21 nt |
| RAG-576 | SEQ ID NO: 237 | GGUGUCUAGGUGCUCCAGG[dT][dT] | 21 nt |
| | SEQ ID NO: 238 | CCUGGAGCACCUAGACACC[dT][dT] | 21 nt |
| RAG-577 | SEQ ID NO: 239 | GGGUGUCUAGGUGCUCCAG[dT][dT] | 21 nt |
| | SEQ ID NO: 240 | CUGGAGCACCUAGACACCC[dT] [dT] | 21 nt |
| RAG-578 | SEQ ID NO: 241 | GGGGUGUCUAGGUGCUCCA[dT][dT] | 21 nt |
| | SEQ ID NO: 242 | UGGAGCACCUAGACACCCC[dT] [dT] | 21 nt |
| RAG-579 | SEQ ID NO: 243 | UGGGGUGUCUAGGUGCUCC[dT][dT] | 21 nt |
| | SEQ ID NO: 244 | GGAGCACCUAGACACCCCA[dT] [dT] | 21 nt |
| RAG-580 | SEQ ID NO: 245 | UUGGGGUGUCUAGGUGCUC[dT] [dT] | 21 nt |
| | SEQ ID NO: 246 | GAGCACCUAGACACCCCAA[dT] [dT] | 21 nt |
| RAG-583 | SEQ ID NO: 247 | UUGUUGGGGUGUCUAGGUG[dT][dT] | 21 nt |
| | SEQ ID NO: 248 | CACCUAGACACCCCAACAA[dT] [dT] | 21 nt |
| RAG-584 | SEQ ID NO: 249 | UUUGUUGGGGUGUCUAGGU[dT][dT] | 21 nt |
| | SEQ ID NO: 250 | ACCUAGACACCCCAACAAA[dT] [dT] | 21 nt |
| RAG-585 | SEQ ID NO: 251 | CUUUGUUGGGGUGUCUAGG[dT][dT] | 21 nt |
| | SEQ ID NO: 252 | CCUAGACACCCCAACAAAG[dT] [dT] | 21 nt |
| **Hotspot 2** | | | |
| RAG-650 | SEQ ID NO: 253 | AGUGGACCUCAAUUUCCUC[dT] [dT] | 21 nt |
| | SEQ ID NO: 254 | GAGGAAAUUGAGGUCCACU[dT][dT] | 21 nt |
| RAG-651 | SEQ ID NO: 255 | CAGUGGACCUCAAUUUCCU[dT][dT] | 21 nt |
| | SEQ ID NO: 256 | AGGAAAUUGAGGUCCACUG[dT] [dT] | 21 nt |
| RAG-652 | SEQ ID NO: 257 | UCAGUGGACCUCAAUUUCC[dT][dT] | 21 nt |
| | SEQ ID NO: 258 | GGAAAUUGAGGUCCACUGA[dT] [dT] | 21 nt |
| RAG-653 | SEQ ID NO: 259 | UUCAGUGGACCUCAAUUUC[dT][dT] | 21 nt |
| | SEQ ID NO: 260 | GAAAUUGAGGUCCACUGAA[dT] [dT] | 21 nt |
| RAG-654 | SEQ ID NO: 261 | GUUCAGUGGACCUCAAUUU[dT][dT] | 21 nt |
| | SEQ ID NO: 262 | AAAUUGAGGUCCACUGAAC[dT] [dT] | 21 nt |
| RAG-655 | SEQ ID NO: 263 | AGUUCAGUGGACCUCAAUU[dT][dT] | 21 nt |
| | SEQ ID NO: 264 | AAUUGAGGUCCACUGAACU[dT][dT] | 21 nt |
| RAG-656 | SEQ ID NO: 265 | AAGUUCAGUGGACCUCAAU[dT][dT] | 21 nt |
| | SEQ ID NO: 266 | AUUGAGGUCCACUGAACUU[dT][dT] | 21 nt |
| RAG-657 | SEQ ID NO: 267 | UAAGUUCAGUGGACCUCAA[dT][dT] | 21 nt |
| | SEQ ID NO: 268 | UUGAGGUCCACUGAACUUA[dT] [dT] | 21 nt |
| RAG-658 | SEQ ID NO: 269 | UUAAGUUCAGUGGACCUCA[dT] [dT] | 21 nt |
| | SEQ ID NO: 270 | UGAGGUCCACUGAACUUAA[dT] [dT] | 21 nt |
| RAG-659 | SEQ ID NO: 271 | CUUAAGUUCAGUGGACCUC[dT][dT] | 21 nt |
| | SEQ ID NO: 272 | GAGGUCCACUGAACUUAAG[dT] [dT] | 21 nt |
| RAG-660 | SEQ ID NO: 273 | ACUUAAGUUCAGUGGACCU[dT][dT] | 21 nt |
| | SEQ ID NO: 274 | AGGUCCACUGAACUUAAGU[dT][dT] | 21 nt |
| RAG-661 | SEQ ID NO: 275 | UACUUAAGUUCAGUGGACC[dT][dT] | 21 nt |
| | SEQ ID NO: 276 | GGUCCACUGAACUUAAGUA[dT][dT] | 21 nt |
| RAG-662 | SEQ ID NO: 277 | AUACUUAAGUUCAGUGGAC[dT] [dT] | 21 nt |
| | SEQ ID NO: 278 | GUCCACUGAACUUAAGUAU[dT][dT] | 21 nt |
| RAG-682 | SEQ ID NO: 279 | GUGACAAUCAACAACUUUG[dT] [dT] | 21 nt |
| | SEQ ID NO: 280 | CAAAGUUGUUGAUUGUCAC[dT][dT] | 21 nt |
| RAG-685 | SEQ ID NO: 281 | CAUGUGACAAUCAACAACU[dT][dT] | 21 nt |
| | SEQ ID NO: 282 | AGUUGUUGAUUGUCACAUG[dT] [dT] | 21 nt |
| RAG-686 | SEQ ID NO: 283 | GCAUGUGACAAUCAACAAC[dT][dT] | 21 nt |
| | SEQ ID NO: 284 | GUUGUUGAUUGUCACAUGC[dT] [dT] | 21 nt |
| RAG-687 | SEQ ID NO: 285 | AGCAUGUGACAAUCAACAA[dT] [dT] | 21 nt |
| | SEQ ID NO: 286 | UUGUUGAUUGUCACAUGCU[dT][dT] | 21 nt |
| RAG-688 | SEQ ID NO: 287 | AAGCAUGUGACAAUCAACA[dT][dT] | 21 nt |
| | SEQ ID NO: 288 | UGUUGAUUGUCACAUGCUU[dT] [dT] | 21 nt |
| RAG-689 | SEQ ID NO: 289 | GAAGCAUGUGACAAUCAAC[dT][dT] | 21 nt |
| | SEQ ID NO: 290 | GUUGAUUGUCACAUGCUUC[dT][dT] | 21 nt |
| RAG-690 | SEQ ID NO: 291 | GGAAGCAUGUGACAAUCAA[dT] [dT] | 21 nt |
| | SEQ ID NO: 292 | UUGAUUGUCACAUGCUUCC[dT][dT] | 21 nt |
| RAG-691 | SEQ ID NO: 293 | CGGAAGCAUGUGACAAUCA[dT] [dT] | 21 nt |
| | SEQ ID NO: 294 | UGAUUGUCACAUGCUUCCG[dT] [dT] | 21 nt |
| RAG-692 | SEQ ID NO: 295 | CCGGAAGCAUGUGACAAUC[dT][dT] | 21 nt |
| | SEQ ID NO: 296 | GAUUGUCACAUGCUUCCGG[dT][dT] | 21 nt |
| RAG-693 | SEQ ID NO: 297 | CCCGGAAGCAUGUGACAAU[dT][dT] | 21 nt |
| | SEQ ID NO: 298 | AUUGUCACAUGCUUCCGGG[dT][dT] | 21 nt |
| RAG-694 | SEQ ID NO: 299 | UCCCGGAAGCAUGUGACAA[dT] [dT] | 21 nt |
| | SEQ ID NO: 300 | UUGUCACAUGCUUCCGGGA[dT][dT] | 21 nt |
| RAG-695 | SEQ ID NO: 301 | UUCCCGGAAGCAUGUGACA[dT] [dT] | 21 nt |
| | SEQ ID NO: 302 | UGUCACAUGCUUCCGGGAA[dT][dT] | 21 nt |
| RAG-696 | SEQ ID NO: 303 | CUUCCCGGAAGCAUGUGAC[dT][dT] | 21 nt |
| | SEQ ID NO: 304 | GUCACAUGCUUCCGGGAAG[dT][dT] | 21 nt |
| RAG-697 | SEQ ID NO: 305 | CCUUCCCGGAAGCAUGUGA[dT] [dT] | 21 nt |
| | SEQ ID NO: 306 | UCACAUGCUUCCGGGAAGG[dT][dT] | 21 nt |
| RAG-698 | SEQ ID NO: 307 | UCCUUCCCGGAAGCAUGUG[dT] [dT] | 21 nt |
| | SEQ ID NO: 308 | CACAUGCUUCCGGGAAGGA[dT][dT] | 21 nt |
| RAG-699 | SEQ ID NO: 309 | CUCCUUCCCGGAAGCAUGU[dT][dT] | 21 nt |
| | SEQ ID NO: 310 | ACAUGCUUCCGGGAAGGAG[dT] [dT] | 21 nt |
| RAG-700 | SEQ ID NO: 311 | CCUCCUUCCCGGAAGCAUG[dT] [dT] | 21 nt |
| | SEQ ID NO: 312 | CAUGCUUCCGGGAAGGAGG[dT][dT] | 21 nt |
| RAG-701 | SEQ ID NO: 313 | CCCUCCUUCCCGGAAGCAU[dT] [dT] | 21 nt |
| | SEQ ID NO: 314 | AUGCUUCCGGGAAGGAGGG[dT] [dT] | 21 nt |
| RAG-702 | SEQ ID NO: 315 | UCCCUCCUUCCCGGAAGCA[dT] [dT] | 21 nt |
| | SEQ ID NO: 316 | UGCUUCCGGGAAGGAGGGA[dT][dT] | 21 nt |
| RAG-704 | SEQ ID NO: 317 | AUUCCCUCCUUCCCGGAAG[dT][dT] | 21 nt |
| | SEQ ID NO: 318 | CUUCCGGGAAGGAGGGAAU[dT][dT] | 21 nt |
| RAG-705 | SEQ ID NO: 319 | AAUUCCCUCCUUCCCGGAA[dT] [dT] | 21 nt |
| | SEQ ID NO: 320 | UUCCGGGAAGGAGGGAAUU[dT][dT] | 21 nt |
| RAG-710 | SEQ ID NO: 321 | UCUCCAAUUCCCUCCUUCC[dT] [dT] | 21 nt |
| | SEQ ID NO: 322 | GGAAGGAGGGAAUUGGAGA[dT][dT] | 21 nt |
| RAG-711 | SEQ ID NO: 323 | CUCUCCAAUUCCCUCCUUC[dT][dT] | 21 nt |
| | SEQ ID NO: 324 | GAAGGAGGGAAUUGGAGAG[dT] [dT] | 21 nt |
| RAG-712 | SEQ ID NO: 325 | UCUCUCCAAUUCCCUCCUU[dT][dT] | 21 nt |
| | SEQ ID NO: 326 | AAGGAGGGAAUUGGAGAGA[dT][dT] | 21 nt |
| RAG-713 | SEQ ID NO: 327 | GUCUCUCCAAUUCCCUCCU[dT][dT] | 21 nt |
| | SEQ ID NO: 328 | AGGAGGGAAUUGGAGAGAC[dT] [dT] | 21 nt |
| RAG-714 | SEQ ID NO: 329 | AGUCUCUCCAAUUCCCUCC[dT][dT] | 21 nt |
| | SEQ ID NO: 330 | GGAGGGAAUUGGAGAGACU[dT][dT] | 21 nt |
| RAG-715 | SEQ ID NO: 331 | UAGUCUCUCCAAUUCCCUC[dT] [dT] | 21 nt |
| | SEQ ID NO: 332 | GAGGGAAUUGGAGAGACUA[dT][dT] | 21 nt |
| RAG-716 | SEQ ID NO: 333 | GUAGUCUCUCCAAUUCCCU[dT][dT] | 21 nt |
| | SEQ ID NO: 334 | AGGGAAUUGGAGAGACUAC[dT][dT] | 21 nt |
| RAG-717 | SEQ ID NO: 335 | GGUAGUCUCUCCAAUUCCC[dT][dT] | 21 nt |
| | SEQ ID NO: 336 | GGGAAUUGGAGAGACUACC[dT] [dT] | 21 nt |

| **Hotspot 1** | | | |
|---|---|---|---|
| RAG-820 | SEQ ID NO: 337 | UGCAACCACAGGGAUUUCU[dT][dT] | 21 nt |
| | SEQ ID NO: 338 | AGAAAUCCCUGUGGUUGCA[dT] [dT] | 21 nt |
| RAG-821 | SEQ ID NO: 339 | CUGCAACCACAGGGAUUUC[dT][dT] | 21 nt |
| | SEQ ID NO: 340 | GAAAUCCCUGUGGUUGCAG[dT] [dT] | 21 nt |
| RAG-822 | SEQ ID NO: 341 | GCUGCAACCACAGGGAUUU[dT][dT] | 21 nt |
| | SEQ ID NO: 342 | AAAUCCCUGUGGUUGCAGC[dT] [dT] | 21 nt |
| RAG-823 | SEQ ID NO: 343 | UGCUGCAACCACAGGGAUU[dT][dT] | 21 nt |
| | SEQ ID NO: 344 | AAUCCCUGUGGUUGCAGCA[dT] [dT] | 21 nt |
| RAG-824 | SEQ ID NO: 345 | CUGCUGCAACCACAGGGAU[dT][dT] | 21 nt |
| | SEQ ID NO: 346 | AUCCCUGUGGUUGCAGCAG[dT][dT] | 21 nt |
| RAG-825 | SEQ ID NO: 347 | GCUGCUGCAACCACAGGGA[dT][dT] | 21 nt |
| | SEQ ID NO: 348 | UCCCUGUGGUUGCAGCAGC[dT] [dT] | 21 nt |
| RAG-826 | SEQ ID NO: 349 | AGCUGCUGCAACCACAGGG[dT][dT] | 21 nt |
| | SEQ ID NO: 350 | CCCUGUGGUUGCAGCAGCU[dT][dT] | 21 nt |
| RAG-828 | SEQ ID NO: 351 | AAAGCUGCUGCAACCACAG[dT][dT] | 21 nt |
| | SEQ ID NO: 352 | CUGUGGUUGCAGCAGCUUU[dT][dT] | 21 nt |
| RAG-829 | SEQ ID NO: 353 | CAAAGCUGCUGCAACCACA[dT][dT] | 21 nt |
| | SEQ ID NO: 354 | UGUGGUUGCAGCAGCUUUG[dT] [dT] | 21 nt |
| RAG-830 | SEQ ID NO: 355 | ACAAAGCUGCUGCAACCAC[dT] [dT] | 21 nt |
| | SEQ ID NO: 356 | GUGGUUGCAGCAGCUUUGU[dT][dT] | 21 nt |
| RAG-831 | SEQ ID NO: 357 | AACAAAGCUGCUGCAACCA[dT] [dT] | 21 nt |
| | SEQ ID NO: 358 | UGGUUGCAGCAGCUUUGUU[dT][dT] | 21 nt |
| RAG-832 | SEQ ID NO: 359 | CAACAAAGCUGCUGCAACC[dT][dT] | 21 nt |
| | SEQ ID NO: 360 | GGUUGCAGCAGCUUUGUUG[dT][dT] | 21 nt |
| RAG-833 | SEQ ID NO: 361 | CCAACAAAGCUGCUGCAAC[dT][dT] | 21 nt |
| | SEQ ID NO: 362 | GUUGCAGCAGCUUUGUUGG[dT][dT] | 21 nt |
| RAG-834 | SEQ ID NO: 363 | GCCAACAAAGCUGCUGCAA[dT][dT] | 21 nt |
| | SEQ ID NO: 364 | UUGCAGCAGCUUUGUUGGC[dT][dT] | 21 nt |
| RAG-835 | SEQ ID NO: 365 | GGCCAACAAAGCUGCUGCA[dT] [dT] | 21 nt |
| | SEQ ID NO: 366 | UGCAGCAGCUUUGUUGGCC[dT][dT] | 21 nt |
| RAG-836 | SEQ ID NO: 367 | UGGCCAACAAAGCUGCUGC[dT][dT] | 21 nt |
| | SEQ ID NO: 368 | GCAGCAGCUUUGUUGGCCA[dT][dT] | 21 nt |
| RAG-837 | SEQ ID NO: 369 | CUGGCCAACAAAGCUGCUG[dT][dT] | 21 nt |
| | SEQ ID NO: 370 | CAGCAGCUUUGUUGGCCAG[dT][dT] | 21 nt |
| RAG-838 | SEQ ID NO: 371 | CCUGGCCAACAAAGCUGCU[dT][dT] | 21 nt |
| | SEQ ID NO: 372 | AGCAGCUUUGUUGGCCAGG[dT] [dT] | 21 nt |
| RAG-840 | SEQ ID NO: 373 | UUCCUGGCCAACAAAGCUG[dT][dT] | 21 nt |
| | SEQ ID NO: 374 | CAGCUUUGUUGGCCAGGAA[dT][dT] | 21 nt |
| RAG-841 | SEQ ID NO: 375 | CUUCCUGGCCAACAAAGCU[dT][dT] | 21 nt |
| | SEQ ID NO: 376 | AGCUUUGUUGGCCAGGAAG[dT] [dT] | 21 nt |
| RAG-843 | SEQ ID NO: 377 | CCCUUCCUGGCCAACAAAG[dT][dT] | 21 nt |
| | SEQ ID NO: 378 | CUUUGUUGGCCAGGAAGGG[dT][dT] | 21 nt |
| RAG-844 | SEQ ID NO: 379 | CCCCUUCCUGGCCAACAAA[dT][dT] | 21 nt |
| | SEQ ID NO: 380 | UUUGUUGGCCAGGAAGGGG[dT][dT] | 21 nt |
| RAG-845 | SEQ ID NO: 381 | UCCCCUUCCUGGCCAACAA[dT] [dT] | 21 nt |
| | SEQ ID NO: 382 | UUGUUGGCCAGGAAGGGGA[dT][dT] | 21 nt |
| RAG-846 | SEQ ID NO: 383 | CUCCCCUUCCUGGCCAACA[dT][dT] | 21 nt |
| | SEQ ID NO: 384 | UGUUGGCCAGGAAGGGGAG[dT][dT] | 21 nt |
| RAG-848 | SEQ ID NO: 385 | UCCUCCCCUUCCUGGCCAA[dT] [dT] | 21 nt |
| | SEQ ID NO: 386 | UUGGCCAGGAAGGGGAGGA[dT] [dT] | 21 nt |
| RAG-849 | SEQ ID NO: 387 | AUCCUCCCCUUCCUGGCCA[dT] [dT] | 21 nt |
| | SEQ ID NO: 388 | UGGCCAGGAAGGGGAGGAU[dT][dT] | 21 nt |
| RAG-853 | SEQ ID NO: 389 | UCAAAUCCUCCCCUUCCUG[dT] [dT] | 21 nt |
| | SEQ ID NO: 390 | CAGGAAGGGGAGGAUUUGA[dT] [dT] | 21 nt |
| RAG-854 | SEQ ID NO: 391 | GUCAAAUCCUCCCCUUCCU[dT][dT] | 21 nt |
| | SEQ ID NO: 392 | AGGAAGGGGAGGAUUUGAC[dT] [dT] | 21 nt |
| RAG-855 | SEQ ID NO: 393 | CGUCAAAUCCUCCCCUUCC[dT][dT] | 21 nt |
| | SEQ ID NO: 394 | GGAAGGGGAGGAUUUGACG[dT] [dT] | 21 nt |
| RAG-856 | SEQ ID NO: 395 | UCGUCAAAUCCUCCCCUUC[dT] [dT] | 21 nt |
| | SEQ ID NO: 396 | GAAGGGGAGGAUUUGACGA[dT] [dT] | 21 nt |
| RAG-857 | SEQ ID NO: 397 | CUCGUCAAAUCCUCCCCUU[dT] [dT] | 21 nt |
| | SEQ ID NO: 398 | AAGGGGAGGAUUUGACGAG[dT] [dT] | 21 nt |
| RAG-858 | SEQ ID NO: 399 | ACUCGUCAAAUCCUCCCCU[dT] [dT] | 21 nt |
| | SEQ ID NO: 400 | AGGGGAGGAUUUGACGAGU[dT][dT] | 21 nt |
| RAG-860 | SEQ ID NO: 401 | UCACUCGUCAAAUCCUCCC[dT][dT] | 21 nt |
| | SEQ ID NO: 402 | GGGAGGAUUUGACGAGUGA[dT] [dT] | 21 nt |
| RAG-861 | SEQ ID NO: 403 | CUCACUCGUCAAAUCCUCC[dT][dT] | 21 nt |
| | SEQ ID NO: 404 | GGAGGAUUUGACGAGUGAG[dT][dT] | 21 nt |
| RAG-862 | SEQ ID NO: 405 | ACUCACUCGUCAAAUCCUC[dT][dT] | 21 nt |
| | SEQ ID NO: 406 | GAGGAUUUGACGAGUGAGU[dT][dT] | 21 nt |
| RAG-864 | SEQ ID NO: 407 | CAACUCACUCGUCAAAUCC[dT][dT] | 21 nt |
| | SEQ ID NO: 408 | GGAUUUGACGAGUGAGUUG[dT][dT] | 21 nt |
| RAG-865 | SEQ ID NO: 409 | ACAACUCACUCGUCAAAUC[dT][dT] | 21 nt |
| | SEQ ID NO: 410 | GAUUUGACGAGUGAGUUGU[dT][dT] | 21 nt |
| RAG-866 | SEQ ID NO: 411 | GACAACUCACUCGUCAAAU[dT][dT] | 21 nt |
| | SEQ ID NO: 412 | AUUUGACGAGUGAGUUGUC[dT] [dT] | 21 nt |
| RAG-867 | SEQ ID NO: 413 | AGACAACUCACUCGUCAAA[dT] [dT] | 21 nt |
| | SEQ ID NO: 414 | UUUGACGAGUGAGUUGUCU[dT][dT] | 21 nt |
| RAG-868 | SEQ ID NO: 415 | CAGACAACUCACUCGUCAA[dT][dT] | 21 nt |
| | SEQ ID NO: 416 | UUGACGAGUGAGUUGUCUG[dT][dT] | 21 nt |
| RAG-869 | SEQ ID NO: 417 | ACAGACAACUCACUCGUCA[dT][dT] | 21 nt |
| | SEQ ID NO: 418 | UGACGAGUGAGUUGUCUGU[dT][dT] | 21 nt |
| RAG-870 | SEQ ID NO: 419 | GACAGACAACUCACUCGUC[dT][dT] | 21 nt |
| | SEQ ID NO: 420 | GACGAGUGAGUUGUCUGUC[dT][dT] | 21 nt |
| RAG-871 | SEQ ID NO: 421 | AGACAGACAACUCACUCGU[dT][dT] | 21 nt |
| | SEQ ID NO: 422 | ACGAGUGAGUUGUCUGUCU[dT][dT] | 21 nt |
| RAG-872 | SEQ ID NO: 423 | GAGACAGACAACUCACUCG[dT][dT] | 21 nt |
| | SEQ ID NO: 424 | CGAGUGAGUUGUCUGUCUC[dT][dT] | 21 nt |
| RAG-873 | SEQ ID NO: 425 | GGAGACAGACAACUCACUC[dT] [dT] | 21 nt |
| | SEQ ID NO: 426 | GAGUGAGUUGUCUGUCUCC[dT][dT] | 21 nt |
| RAG-874 | SEQ ID NO: 427 | AGGAGACAGACAACUCACU[dT][dT] | 21 nt |
| | SEQ ID NO: 428 | AGUGAGUUGUCUGUCUCCU[dT][dT] | 21 nt |
| RAG-875 | SEQ ID NO: 429 | CAGGAGACAGACAACUCAC[dT] [dT] | 21 nt |
| | SEQ ID NO: 430 | GUGAGUUGUCUGUCUCCUG[dT] [dT] | 21 nt |
| RAG-876 | SEQ ID NO: 431 | UCAGGAGACAGACAACUCA[dT] [dT] | 21 nt |
| | SEQ ID NO: 432 | UGAGUUGUCUGUCUCCUGA[dT][dT] | 21 nt |
| RAG-877 | SEQ ID NO: 433 | UUCAGGAGACAGACAACUC[dT][dT] | 21 nt |
| | SEQ ID NO: 434 | GAGUUGUCUGUCUCCUGAA[dT][dT] | 21 nt |
| RAG-878 | SEQ ID NO: 435 | AUUCAGGAGACAGACAACU[dT][dT] | 21 nt |
| | SEQ ID NO: 436 | AGUUGUCUGUCUCCUGAAU[dT][dT] | 21 nt |
| RAG-879 | SEQ ID NO: 437 | UAUUCAGGAGACAGACAAC[dT] [dT] | 21 nt |
| | SEQ ID NO: 438 | GUUGUCUGUCUCCUGAAUA[dT][dT] | 21 nt |
| RAG-880 | SEQ ID NO: 439 | GUAUUCAGGAGACAGACAA[dT] [dT] | 21 nt |
| | SEQ ID NO: 440 | UUGUCUGUCUCCUGAAUAC[dT] [dT] | 21 nt |
| RAG-881 | SEQ ID NO: 441 | AGUAUUCAGGAGACAGACA[dT] [dT] | 21 nt |
| | SEQ ID NO: 442 | UGUCUGUCUCCUGAAUACU[dT][dT] | 21 nt |
| RAG-882 | SEQ ID NO: 443 | GAGUAUUCAGGAGACAGAC[dT] [dT] | 21 nt |
| | SEQ ID NO: 444 | GUCUGUCUCCUGAAUACUC[dT] [dT] | 21 nt |
| RAG-883 | SEQ ID NO: 445 | GGAGUAUUCAGGAGACAGA[dT] [dT] | 21 nt |
| | SEQ ID NO: 446 | UCUGUCUCCUGAAUACUCC[dT][dT] | 21 nt |
| RAG-884 | SEQ ID NO: 447 | GGGAGUAUUCAGGAGACAG[dT] [dT] | 21 nt |
| | SEQ ID NO: 448 | CUGUCUCCUGAAUACUCCC[dT][dT] | 21 nt |
| RAG-885 | SEQ ID NO: 449 | GGGGAGUAUUCAGGAGACA[dT] [dT] | 21 nt |
| | SEQ ID NO: 450 | UGUCUCCUGAAUACUCCCC[dT] [dT] | 21 nt |
| RAG-886 | SEQ ID NO: 451 | UGGGGAGUAUUCAGGAGAC[dT][dT] | 21 nt |
| | SEQ ID NO: 452 | GUCUCCUGAAUACUCCCCA[dT] [dT] | 21 nt |
| RAG-887 | SEQ ID NO: 453 | GUGGGGAGUAUUCAGGAGA[dT] [dT] | 21 nt |
| | SEQ ID NO: 454 | UCUCCUGAAU ACUCCCCAC[dT] [dT] | 21 nt |
| RAG-888 | SEQ ID NO: 455 | UGUGGGGAGUAUUCAGGAG[dT][dT] | 21 nt |
| | SEQ ID NO: 456 | CUCCUGAAUACUCCCCACA[dT][dT] | 21 nt |
| RAG-889 | SEQ ID NO: 457 | AUGUGGGGAGUAUUCAGGA[dT][dT] | 21 nt |
| | SEQ ID NO: 458 | UCCUGAAUACUCCCCACAU[dT] [dT] | 21 nt |
| RAG-890 | SEQ ID NO: 459 | UAUGUGGGGAGUAUUCAGG[dT] [dT] | 21 nt |
| | SEQ ID NO: 460 | CCUGAAUACUCCCCACAUA[dT] [dT] | 21 nt |
| RAG-891 | SEQ ID NO: 461 | CUAUGUGGGGAGUAUUCAG[dT][dT] | 21 nt |
| | SEQ ID NO: 462 | CUGAAUACUCCCCACAUAG[dT] [dT] | 21 nt |
| RAG-892 | SEQ ID NO: 463 | GCUAUGUGGGGAGUAUUCA[dT][dT] | 21 nt |
| | SEQ ID NO: 464 | UGAAUACUCCCCACAUAGC[dT] [dT] | 21 nt |
| RAG-893 | SEQ ID NO: 465 | GGCUAUGUGGGGAGUAUUC[dT] [dT] | 21 nt |
| | SEQ ID NO: 466 | GAAUACUCCCCACAUAGCC[dT] [dT] | 21 nt |
| RAG-894 | SEQ ID NO: 467 | GGGCUAUGUGGGGAGUAUU[dT][dT] | 21 nt |
| | SEQ ID NO: 468 | AAUACUCCCCACAUAGCCC[dT] [dT] | 21 nt |

These hotspots include: hotspot 1 having a corresponding target sequence from -893 bp to -801 bp in the p21 promoter sequence, shown as SEQ ID NO: 93, wherein 44 functional saRNAs (Table 3, Fig. 3A) were discovered in this region, comprising RAG-834, RAG-845, RAG-892, RAG-846, RAG-821, RAG-884, RAG-864, RAG-843, RAG-854, RAG-844, RAG-887, RAG-838, RAG-858, RAG-835, RAG-876, RAG-870, RAG-853, RAG-881, RAG-828, RAG-872, RAG-841, RAG-831, RAG-829, RAG-820, RAG-822, RAG-868, RAG-849, RAG-862, RAG-865, RAG-893, RAG-848, RAG-824, RAG-866, RAG-840, RAG-875, RAG-880, RAG-871, RAG-888, RAG-885, RAG-894, RAG-833, RAG-825, RAG-889, and RAG-823;
hotspot 2 (Table 3, Fig. 3B) having a corresponding target sequence from -717 bp to - 632 bp in the p21 promoter sequence, shown as SEQ ID NO: 94, wherein 31 functional saRNAs were discovered in this region, comprising RAG-693, RAG-692, RAG-688, RAG-696, RAG-694, RAG-687, RAG-691, RAG-690, RAG-689, RAG-682, RAG-686, RAG-662, RAG-695, RAG-654, RAG-658, RAG-685, RAG-704, RAG-714, RAG-705, RAG-661, RAG-656, RAG-698, RAG-697, RAG-657, RAG-715, RAG-652, RAG-651, RAG-650, RAG-716, RAG-717, and RAG-711;
hotspot 3 (Table 3, Fig. 3C) having a corresponding target sequence from -585 bp to - 551 bp in the p21 promoter sequence, shown as SEQ ID NO: 95, wherein 9 functional saRNAs were discovered in this region, comprising RAG-580, RAG-577, RAG-569, RAG-576, RAG-570, RAG-574, RAG-585, RAG-579, and RAG-584;
hotspot 4 (Table 3, Fig. 3D) having a corresponding target sequence from -554 bp to - 505 bp in the p21 promoter sequence, shown as SEQ ID NO: 96, wherein 17 functional saRNAs were discovered in this region, comprising RAG-524, RAG-553, RAG-537, RAG-526, RAG-554, RAG-523, RAG-534, RAG-543, RAG-525, RAG-535, RAG-546, RAG-545, RAG-542, RAG-531, RAG-522, RAG-529, and RAG-552;
hotspot 5 (Table 3, Fig. 3E) having a corresponding target sequence from -514 bp to - 485 bp in the p21 promoter sequence, shown as SEQ ID NO: 97, wherein 9 functional saRNAs were discovered in this region, comprising RAG-503, RAG-504, RAG-505, RAG-506, RAG-507, RAG-508, RAG-509, RAG-510, RAG-511, RAG-512, RAG-513, and RAG-514;
hotspot 6 (Table 3, Fig. 3F) having a corresponding target sequence from -442 bp to - 405 bp in the p21 promoter sequence, shown as SEQ ID NO: 98, wherein 12 functional saRNAs were discovered in this region, comprising RAG-427, RAG-430, RAG-431, RAG-423, RAG-425, RAG-433, RAG-435, RAG-434, RAG-439, RAG-426, RAG-428, and RAG-442;
hotspot 7 (Table 3, Fig. 3G) having a corresponding target sequence from -352 bp to - 313 bp in the p21 promoter sequence, shown as SEQ ID NO: 99, wherein 13 functional saRNAs were discovered in this region, comprising RAG-335, RAG-351, RAG-352, RAG-331, RAG-344, RAG-342, RAG-341, RAG-333, RAG-345, RAG-346, RAG-336, RAG-332, and RAG-343;
and hotspot 8 (Table 3, Fig. 3H) having a corresponding target sequence from -325 bp to -260 bp in the p21 promoter sequence, shown as SEQ ID NO: 100, wherein 18 functional saRNAs were discovered in this region, comprising RAG-294, RAG-285, RAG-286, RAG-292, RAG-291, RAG-284, RAG-279, RAG-280, RAG-325, RAG-293, RAG-322, RAG-321, RAG-281, RAG-289, RAG-278, RAG-283, RAG-282, and RAG-295.

In order to verify the QuantiGene 2.0 assay results, the 439 double-stranded RNA molecules were divided into four bins according to their activities in inducing p21 mRNA expression, and 5 double-stranded RNA molecules were randomly selected from each bin and transfected into PC3 cells at a concentration of 10 nM. 72 hours after transfection, total cellular RNA was extracted from the transfected cells and reverse transcribed into cDNA which was amplified by RT-qPCR to determine p21 mRNA level. p21 mRNA expression levels for cells transfected with each of the saRNAs determined by the two methods showed a significant correlation (R² = 0.82) (Fig. 4). All selected functional saRNAs obtained through the QuantiGene 2.0 method were verified as real functional saRNAs by RT-qPCR, and some of them exhibited even a stronger p21 mRNA induction ability by the RT-qPCR (Table 4).

**Table 4: Verification for QuantiGene 2.0 method**

| **Bin** | **Title** | **Relative p21 mRNA level** | |
|---|---|---|---|
| | | Quantigene 2.0 | RT-qPCR |
| bin-1 | RAG-693 | 8.12 | 48.20 |
| | RAG-834 | 8.07 | 9.64 |
| | RAG-692 | 7.69 | 29.53 |
| | RAG-845 | 6.67 | 7.15 |
| | RAG-688 | 6.55 | 42.91 |
| bin-2 | RAG-531 | 2.06 | 3.11 |
| | RAG-705 | 2.05 | 11.33 |
| | RAG-322 | 2.04 | 7.53 |
| | RAG-840 | 2.03 | 7.01 |
| | RAG-741 | 2.02 | 5.45 |
| bin-3 | RAG-883 | 1.00 | 1.76 |
| | RAG-177 | 1.00 | 0.31 |
| | RAG-530 | 1.00 | 1.41 |
| | RAG-879 | 1.00 | 0.98 |
| | RAG-527 | 0.99 | 1.06 |
| bin-4 | RAG-830 | 0.72 | 0.45 |
| | RAG-419 | 0.71 | 0.41 |
| | RAG-420 | 0.71 | 0.93 |
| | RAG-700 | 0.69 | 0.32 |
| | RAG-589 | 0.66 | 0.80 |

Taken together, the above data indicates that saRNAs can be designed to target selected regions in the p21 promoter to induce p21 expression with certain regions being more sensitive and containing higher percentages of functional saRNA targets.

### Example 2: saRNAs induce p21 mRNA expression and inhibit cancer cell proliferation

In order to further evaluate the effect of p21 saRNAs in inducing p21 mRNA expression and suppressing cancer cell proliferation, the saRNAs (RAG1-431, RAG1-553, and RAG1-688) screened by QuantiGene 2.0 were transfected into cancer cell lines including KU-7 (bladder cancer), HCT116 (colon cancer), and HepG2 (hepatocellular carcinoma). The result showed that in all the aforementioned cell lines, all saRNAs can induce at least a two-fold change in the p21 mRNA expression levels and suppress cell proliferation, indicating functional activation of p21 protein. Specifically, RAG1-431, RAG1-553, and RAG1-688 were individually transfected into KU-7 cells, caused a 14.0-, 36.9- and 31.9-fold change in the mRNA expression of p21, and exhibited a 71.7%, 60.7% and 67.4% cell survival rate respectively relative to blank control (Mock) (Fig. 5). RAG1-431, RAG1-553, and RAG1-688 were transfected into the HCT116 cells, resulted in a 2.3-, 3.5-, and 2.4-fold change in the mRNA expression of p21, and exhibited a survival rate of 45.3%, 22.5% and 38.5% respectively relative to the blank control (Mock) (Fig. 6). RAG1-431, RAG1-553, and RAG1-688 were transfected into the HepG2 cells, resulted in a 2.2-, 3.3- and 2.0-fold change in the mRNA expression of p21, and exhibited a survival rate of 76.7%, 64.9%, and 79.9% relative to the blank control (Mock) (Fig. 7).

### Incorporation by reference

All disclosures of each patent literature and scientific literature cited herein are incorporated herein by reference for all purposes.

### Equivalence

The present invention can be implemented in other specific forms without departing from its fundamental characteristics. Therefore, the aforementioned examples shall be considered as illustrative rather than restrictive to the present invention described herein. The scope of the present invention is represented by the appended claims rather than the above specification, and is intended to cover all changes falling into the meanings and scopes of equivalents of the claims.

## Claims

1. A saRNA, wherein one strand of the saRNA has at least 75% homology or complementarity with any continuous fragment of 16 to 35 nucleotides in length in a nucleic acid sequence selected from the group consisting of SEQ ID NOs: 5, 6, 7, 8, 9, 10, 11, and 12, and wherein the saRNA activates or upregulates the expression of p21 by targeting a sequence of a human p21 promoter.

2. The saRNA of claim 1, wherein the saRNA comprises a sense nucleic acid strand and an antisense nucleic acid strand, the sense nucleic acid strand and the antisense nucleic acid strand contain complementary regions capable of forming a double-stranded nucleic acid structure, and the sense nucleic acid strand or the antisense nucleic acid strand has more than 75%, more than 80%, more than 90%, more than 95%, more than 99%, or 100% homology with any continuous fragment of 16 to 35 nucleotides in length in a sequence of a human p21 promoter.

3. The saRNA of claim 2, wherein the sense nucleic acid strand and the antisense nucleic acid strand are on two different nucleic acid strands.

4. The saRNA of claim 2, wherein the sense nucleic acid strand and the antisense nucleic acid strand are on the same nucleic acid strand, forming a hairpin single-stranded nucleic acid molecule, wherein the complementary regions of the sense nucleic acid strand and the antisense nucleic acid strand form a double-stranded nucleic acid structure.

5. The saRNA of claim 3, wherein at least one strand of the saRNA has a 3' overhang of 0 to 6 nucleotides in length.

6. The saRNA of claim 5, wherein both strands of the saRNA have a 3' overhang of 2 to 3 nucleotides in length.

7. The saRNA of any one of claims 2 to 6, wherein the sense nucleic acid strand or the antisense nucleic acid strand is 16 to 35 nucleotides in length.

8. The saRNA of claim 1, wherein the sense nucleic acid strand or the antisense nucleic acid strand has at least 75% homology with a nucleic acid sequence selected from the group consisting of SEQ ID NOs: 13-30, 35-46, 59-62, 67-74, 77-80, 85-96, 103-108, 111-118, 121-132, 139-140, 147-180, 185-186, 189-190, 195-198, 201-202, 209-212, 215-218, 225-240, 243-246, 249-258, 261-262, 265-270, 275-280, 283-300, 303-308, 317-320, 323-324, 329-348, 351-352, 357-358, 361-366, 371-392, 399-400, 405-412, 415-416, 419-424, 429-432, 439-442, 447-450, 453-458, and 463-468.

9. The saRNA of claim 8, wherein the nucleotide sequence of the sense nucleic acid strand or the antisense nucleic acid strand is selected from the group consisting of SEQ ID NOs: 13-30, 35-46, 59-62, 67-74, 77-80, 85-96, 103-108, 111-118, 121-132, 139-140, 147-180, 185-186, 189-190, 195-198, 201-202, 209-212, 215-218, 225-240, 243-246, 249-258, 261-262, 265-270, 275-280, 283-300, 303-308, 317-320, 323-324, 329-348, 351-352, 357-358, 361-366, 371-392, 399-400, 405-412, 415-416, 419-424, 429-432, 439-442, 447-450, 453-458, and 463-468.

10. The saRNA of any one of claims 1 to 9, wherein at least one nucleotide of the saRNA is a chemically modified nucleotide, and the chemical modification is at least one of the following modifications:
(1) modification of a phosphodiester bond connecting nucleotides in the nucleotide sequence of the saRNA;
(2) modification of 2'-OH of ribose in the nucleotide sequence of the saRNA;
(3) modification of a base in the nucleotide sequence of the saRNA; or
(4) at least one nucleotide in the nucleotide sequence of the small activating nucleic acid molecule being a locked nucleic acid.

11. The saRNA of any one of claims 1 to 10, wherein the expression of p21 is activated or upregulated by at least 10%.

12. A use of the saRNA of any one of claims 1 to 11 in the preparation of a formulation for activating or upregulating the expression of p21 in a cell.

13. The use of claim 12, wherein the saRNA is introduced into the cell directly.

14. The use of claim 13, wherein the cell is a mammalian cell, preferably a human cell, and more preferably a human tumor cell.

15. The use of claim 14, wherein the cell is present in a human body.

16. The use of claim 15, wherein the human body is a patient with a tumor caused by insufficient p21 protein expression, moreover, administration of an effective amount of the small activating nucleic acid molecule can treat the tumor, and the tumor is preferably a bladder cancer, a prostate cancer, a hepatocellular carcinoma, or a colorectal cancer.

17. An isolated p21 saRNA target site, wherein the target site is any continuous 16-35 nucleotide sequence selected from the group consisting of SEQ ID NOs. 5-12.

18. A method for activating or upregulating the expression of p21 in a cell, wherein the method comprises administrating the saRNA of any one of claims 1 to 11 to the cell.

19. The method of claim 18, wherein the saRNA is introduced into the cell directly.

20. The method of claim 18 or 19, wherein the cell is a mammal cell, preferably a human cell, more preferably a human tumor cell, and further more preferably a bladder cancer cell, a prostate cancer cell, a hepatocellular carcinoma cell, or a colorectal cancer cell.

21. A composition comprising the saRNA of any one of claims 1 to 11 and a pharmaceutically acceptable carrier.

22. The composition of claim 21, wherein the pharmaceutically acceptable carrier is a liposome, a macromolecular polymer, or a polypeptide.

23. A use of the saRNA of any one of claims 1 to 11 or the composition of claim 21 or 22 in the preparation of a formulation for activating or upregulating p21 expression, and preferably in the preparation of a formulation for treating a tumor or a benign proliferative lesion, wherein the tumor is more preferably a bladder cancer, a prostate cancer, a hepatocellular carcinoma, or a colorectal cancer.


SEQUENCE LISTING

<110>  Ractigen Therapeutics

<120>  Method for Activating P21 Gene Expression

<130>  065786.11001/2US1

<150>  PCT/CN2019/082126
<151>  2019-04-10

<150>  CN201810316672.1
<151>  2018-04-10

<160>  468

<170>  PatentIn version 3.5

<210>  1
<211>  21
<212>  DNA
<213>  Artificial Sequence

<220>
<223>  GAPDH F Primer

<400>  1
atcaccatct tccaggagcg a                                                21


<210>  2
<211>  21
<212>  DNA
<213>  Artificial Sequence

<220>
<223>  GAPDH R Primer

<400>  2
ttctccatgg tggtgaagac g                                                21


<210>  3
<211>  20
<212>  DNA
<213>  Artificial Sequence

<220>
<223>  CDKN1A F Primer

<400>  3
ggaagaccat gtggacctgt                                                  20


<210>  4
<211>  20
<212>  DNA
<213>  Artificial Sequence

<220>
<223>  CDKN1A R Primer

<400>  4

ggattagggc ttcctcttgg                                                20

<210>  5
<211>  92
<212>  DNA
<213>  Homo sapiens

<400>  5
ggctatgtgg ggagtattca ggagacagac aactcactcg tcaaatcctc cccttcctgg      60

ccaacaaagc tgctgcaacc acagggattt ct                                    92

<210>  6
<211>  86
<212>  DNA
<213>  Homo sapiens

<400>  6
ggtagtctct ccaattccct ccttcccgga agcatgtgac aatcaacaac tttgtatact      60

taagttcagt ggacctcaat ttcctc                                           86

<210>  7
<211>  35
<212>  DNA
<213>  Homo sapiens

<400>  7
ctttgttggg gtgtctaggt gctccaggtg cttct                                 35

<210>  8
<211>  51
<212>  DNA
<213>  Homo sapiens

<400>  8
ttctgggaga ggtgacctag tgagggatca gtgggaatag aggtgatatt g               51

<210>  9
<211>  30
<212>  DNA
<213>  Homo sapiens

<400>  9
aggtgatatt gtggggcttt tctggaaatt                                       30

<210>  10
<211>  38
<212>  DNA
<213>  Homo sapiens

<400>  10
attaatgtca tcctcctgat cttttcagct gcattggg                              38

<210>  11
<211>  40

<212>  DNA
<213>  Homo sapiens

<400>  11
tctaacagtg ctgtgtcctc ctggagagtg ccaactcatt                40


<210>  12
<211>  66
<212>  DNA
<213>  Homo sapiens

<400>  12
gtgccaactc attctccaag taaaaaaagc cagatttgtg gctcacttcg tggggaaatg      60

tgtcca                                                                 66


<210>  13
<211>  21
<212>  DNA
<213>  Artificial Sequence

<220>
<223>  RAG-278

<400>  13
ucguggggaa auguguccat t                                    21


<210>  14
<211>  21
<212>  DNA
<213>  Artificial Sequence

<220>
<223>  RAG-278

<400>  14
uggacacauu uccccacgat t                                    21


<210>  15
<211>  21
<212>  DNA
<213>  Artificial Sequence

<220>
<223>  RAG-279

<400>  15
uucgugggga aaugugucct t                                    21


<210>  16
<211>  21
<212>  DNA
<213>  Artificial Sequence

<220>
<223>  RAG-279

<400>  16

ggacacauuu ccccacgaat t                                                21

<210>  17
<211>  21
<212>  DNA
<213>  Artificial Sequence

<220>
<223>  RAG-280

<400>  17
cuucgugggg aaauguguct t                                                21

<210>  18
<211>  21
<212>  DNA
<213>  Artificial Sequence

<220>
<223>  RAG-280

<400>  18
gacacauuuc cccacgaagt t                                                21

<210>  19
<211>  21
<212>  DNA
<213>  Artificial Sequence

<220>
<223>  RAG-281

<400>  19
acuucguggg gaaaugugut t                                                21

<210>  20
<211>  21
<212>  DNA
<213>  Artificial Sequence

<220>
<223>  RAG-281

<400>  20
acacauuucc ccacgaagut t                                                21

<210>  21
<211>  21
<212>  DNA
<213>  Artificial Sequence

<220>
<223>  RAG-282

<400>  21
cacuucgugg ggaaaugugt t                                                21

<210>  22
<211>  21
<212>  DNA
<213>  Artificial Sequence

<220>
<223>  RAG-282

<400>  22
cacauuuccc cacgaagugt t                                                21


<210>  23
<211>  21
<212>  DNA
<213>  Artificial Sequence

<220>
<223>  RAG-283

<400>  23
ucacuucgug gggaaaugut t                                                21


<210>  24
<211>  21
<212>  DNA
<213>  Artificial Sequence

<220>
<223>  RAG-283

<400>  24
acauuucccc acgaagugat t                                                21


<210>  25
<211>  21
<212>  DNA
<213>  Artificial Sequence

<220>
<223>  RAG-284

<400>  25
cucacuucgu ggggaaaugt t                                                21


<210>  26
<211>  21
<212>  DNA
<213>  Artificial Sequence

<220>
<223>  RAG-284

<400>  26
cauuucccca cgaagugagt t                                                21


<210>  27
<211>  21
<212>  DNA

<213>  Artificial Sequence

<220>
<223>  RAG-285

<400>  27
gcucacuucg uggggaaaut t                                                21


<210>  28
<211>  21
<212>  DNA
<213>  Artificial Sequence

<220>
<223>  RAG-285

<400>  28
auuuccccac gaagugagct t                                                21


<210>  29
<211>  21
<212>  DNA
<213>  Artificial Sequence

<220>
<223>  RAG-286

<400>  29
ggcucacuuc guggggaaat t                                                21


<210>  30
<211>  21
<212>  DNA
<213>  Artificial Sequence

<220>
<223>  RAG-286

<400>  30
uuuccccacg aagugagcct t                                                21


<210>  31
<211>  21
<212>  DNA
<213>  Artificial Sequence

<220>
<223>  RAG-287

<400>  31
uggcucacuu cguggggaat t                                                21


<210>  32
<211>  21
<212>  DNA
<213>  Artificial Sequence

<220>

<223>  RAG-287

<400>  32
uuccccacga agugagccat t                                                21


<210>  33
<211>  21
<212>  DNA
<213>  Artificial Sequence

<220>
<223>  RAG-288

<400>  33
guggcucacu ucguggggat t                                                21


<210>  34
<211>  21
<212>  DNA
<213>  Artificial Sequence

<220>
<223>  RAG-288

<400>  34
uccccacgaa gugagccact t                                                21


<210>  35
<211>  21
<212>  DNA
<213>  Artificial Sequence

<220>
<223>  RAG-289

<400>  35
uguggcucac uucguggggt t                                                21


<210>  36
<211>  21
<212>  DNA
<213>  Artificial Sequence

<220>
<223>  RAG-289

<400>  36
ccccacgaag ugagccacat t                                                21


<210>  37
<211>  21
<212>  DNA
<213>  Artificial Sequence

<220>
<223>  RAG-291

<400>  37

uuuguggcuc acuucguggt t                                                21

<210>  38
<211>  21
<212>  DNA
<213>  Artificial Sequence

<220>
<223>  RAG-291

<400>  38
ccacgaagug agccacaaat t                                                21

<210>  39
<211>  21
<212>  DNA
<213>  Artificial Sequence

<220>
<223>  RAG-292

<400>  39
auuuguggcu cacuucgugt t                                                21

<210>  40
<211>  21
<212>  DNA
<213>  Artificial Sequence

<220>
<223>  RAG-292

<400>  40
cacgaaguga gccacaaaut t                                                21

<210>  41
<211>  21
<212>  DNA
<213>  Artificial Sequence

<220>
<223>  RAG-293

<400>  41
gauuuguggc ucacuucgut t                                                21

<210>  42
<211>  21
<212>  DNA
<213>  Artificial Sequence

<220>
<223>  RAG-293

<400>  42
acgaagugag ccacaaauct t                                                21

<210>  43
<211>  21
<212>  DNA
<213>  Artificial Sequence

<220>
<223>  RAG-294

<400>  43
agauuugugg cucacuucgt t                                                21


<210>  44
<211>  21
<212>  DNA
<213>  Artificial Sequence

<220>
<223>  RAG-294

<400>  44
cgaagugagc cacaaaucut t                                                21


<210>  45
<211>  21
<212>  DNA
<213>  Artificial Sequence

<220>
<223>  RAG-295

<400>  45
cagauuugug gcucacuuct t                                                21


<210>  46
<211>  21
<212>  DNA
<213>  Artificial Sequence

<220>
<223>  RAG-295

<400>  46
gaagugagcc acaaaucugt t                                                21


<210>  47
<211>  21
<212>  DNA
<213>  Artificial Sequence

<220>
<223>  RAG-296

<400>  47
ccagauuugu ggcucacuut t                                                21


<210>  48
<211>  21
<212>  DNA

<213>  Artificial Sequence

<220>
<223>  RAG-296

<400>  48
aagugagcca caaaucuggt t                                                21


<210>  49
<211>  21
<212>  DNA
<213>  Artificial Sequence

<220>
<223>  RAG-297

<400>  49
gccagauuug uggcucacut t                                                21


<210>  50
<211>  21
<212>  DNA
<213>  Artificial Sequence

<220>
<223>  RAG-297

<400>  50
agugagccac aaaucuggct t                                                21


<210>  51
<211>  21
<212>  DNA
<213>  Artificial Sequence

<220>
<223>  RAG-298

<400>  51
agccagauuu guggcucact t                                                21


<210>  52
<211>  21
<212>  DNA
<213>  Artificial Sequence

<220>
<223>  RAG-298

<400>  52
gugagccaca aaucuggcut t                                                21


<210>  53
<211>  21
<212>  DNA
<213>  Artificial Sequence

<220>

<223>  RAG-299

<400>  53
aagccagauu uguggcucat t                                                21


<210>  54
<211>  21
<212>  DNA
<213>  Artificial Sequence

<220>
<223>  RAG-299

<400>  54
ugagccacaa aucuggcuut t                                                21


<210>  55
<211>  21
<212>  DNA
<213>  Artificial Sequence

<220>
<223>  RAG-300

<400>  55
aaagccagau uuguggcuct t                                                21


<210>  56
<211>  21
<212>  DNA
<213>  Artificial Sequence

<220>
<223>  RAG-300

<400>  56
gagccacaaa ucuggcuuut t                                                21


<210>  57
<211>  21
<212>  DNA
<213>  Artificial Sequence

<220>
<223>  RAG-301

<400>  57
aaaagccaga uuuguggcut t                                                21


<210>  58
<211>  21
<212>  DNA
<213>  Artificial Sequence

<220>
<223>  RAG-301

<400>  58

agccacaaau cuggcuuuut t                                                21

<210>  59
<211>  21
<212>  DNA
<213>  Artificial Sequence

<220>
<223>  RAG-321

<400>  59
caacucauuc uccaaguaat t                                                21

<210>  60
<211>  21
<212>  DNA
<213>  Artificial Sequence

<220>
<223>  RAG-321

<400>  60
uuacuuggag aaugaguugt t                                                21

<210>  61
<211>  21
<212>  DNA
<213>  Artificial Sequence

<220>
<223>  RAG-322

<400>  61
ccaacucauu cuccaaguat t                                                21

<210>  62
<211>  21
<212>  DNA
<213>  Artificial Sequence

<220>
<223>  RAG-322

<400>  62
uacuuggaga augaguuggt t                                                21

<210>  63
<211>  21
<212>  DNA
<213>  Artificial Sequence

<220>
<223>  RAG-323

<400>  63
gccaacucau ucuccaagut t                                                21

<210>  64
<211>  21
<212>  DNA
<213>  Artificial Sequence

<220>
<223>  RAG-323

<400>  64
acuuggagaa ugaguuggct t                                                21


<210>  65
<211>  21
<212>  DNA
<213>  Artificial Sequence

<220>
<223>  RAG-324

<400>  65
ugccaacuca uucuccaagt t                                                21


<210>  66
<211>  21
<212>  DNA
<213>  Artificial Sequence

<220>
<223>  RAG-324

<400>  66
cuuggagaau gaguuggcat t                                                21


<210>  67
<211>  21
<212>  DNA
<213>  Artificial Sequence

<220>
<223>  RAG-325

<400>  67
gugccaacuc auucuccaat t                                                21


<210>  68
<211>  21
<212>  DNA
<213>  Artificial Sequence

<220>
<223>  RAG-325

<400>  68
uuggagaaug aguuggcact t                                                21


<210>  69
<211>  21
<212>  DNA

<213>  Artificial Sequence

<220>
<223>  RAG-331

<400>  69
uggagagugc caacucauut t                                                21


<210>  70
<211>  21
<212>  DNA
<213>  Artificial Sequence

<220>
<223>  RAG-331

<400>  70
aaugaguugg cacucuccat t                                                21


<210>  71
<211>  21
<212>  DNA
<213>  Artificial Sequence

<220>
<223>  RAG-332

<400>  71
cuggagagug ccaacucaut t                                                21


<210>  72
<211>  21
<212>  DNA
<213>  Artificial Sequence

<220>
<223>  RAG-332

<400>  72
augaguuggc acucuccagt t                                                21


<210>  73
<211>  21
<212>  DNA
<213>  Artificial Sequence

<220>
<223>  RAG-333

<400>  73
ccuggagagu gccaacucat t                                                21


<210>  74
<211>  21
<212>  DNA
<213>  Artificial Sequence

<220>

<223>  RAG-333

<400>  74
ugaguuggca cucuccaggt t                                                21


<210>  75
<211>  21
<212>  DNA
<213>  Artificial Sequence

<220>
<223>  RAG-334

<400>  75
uccuggagag ugccaacuct t                                                21


<210>  76
<211>  21
<212>  DNA
<213>  Artificial Sequence

<220>
<223>  RAG-334

<400>  76
gaguuggcac ucuccaggat t                                                21


<210>  77
<211>  21
<212>  DNA
<213>  Artificial Sequence

<220>
<223>  RAG-335

<400>  77
cuccuggaga gugccaacut t                                                21


<210>  78
<211>  21
<212>  DNA
<213>  Artificial Sequence

<220>
<223>  RAG-335

<400>  78
aguuggcacu cuccaggagt t                                                21


<210>  79
<211>  21
<212>  DNA
<213>  Artificial Sequence

<220>
<223>  RAG-336

<400>  79

ccuccuggag agugccaact t                                                21

<210>  80
<211>  21
<212>  DNA
<213>  Artificial Sequence

<220>
<223>  RAG-336

<400>  80
guuggcacuc uccaggaggt t                                                21

<210>  81
<211>  21
<212>  DNA
<213>  Artificial Sequence

<220>
<223>  RAG-337

<400>  81
uccuccugga gagugccaat t                                                21

<210>  82
<211>  21
<212>  DNA
<213>  Artificial Sequence

<220>
<223>  RAG-337

<400>  82
uuggcacucu ccaggaggat t                                                21

<210>  83
<211>  21
<212>  DNA
<213>  Artificial Sequence

<220>
<223>  RAG-338

<400>  83
guccuccugg agagugccat t                                                21

<210>  84
<211>  21
<212>  DNA
<213>  Artificial Sequence

<220>
<223>  RAG-338

<400>  84
uggcacucuc caggaggact t                                                21

<210>  85
<211>  21
<212>  DNA
<213>  Artificial Sequence

<220>
<223>  RAG-341

<400>  85
uguguccucc uggagagugt t                                                21


<210>  86
<211>  21
<212>  DNA
<213>  Artificial Sequence

<220>
<223>  RAG-341

<400>  86
cacucuccag gaggacacat t                                                21


<210>  87
<211>  21
<212>  DNA
<213>  Artificial Sequence

<220>
<223>  RAG-342

<400>  87
cuguguccuc cuggagagut t                                                21


<210>  88
<211>  21
<212>  DNA
<213>  Artificial Sequence

<220>
<223>  RAG-342

<400>  88
acucuccagg aggacacagt t                                                21


<210>  89
<211>  21
<212>  DNA
<213>  Artificial Sequence

<220>
<223>  RAG-343

<400>  89
gcuguguccu ccuggagagt t                                                21


<210>  90
<211>  21
<212>  DNA

<213>  Artificial Sequence

<220>
<223>  RAG-343

<400>  90
cucuccagga ggacacagct t                                                21


<210>  91
<211>  21
<212>  DNA
<213>  Artificial Sequence

<220>
<223>  RAG-344

<400>  91
ugcugugucc uccuggagat t                                                21


<210>  92
<211>  21
<212>  DNA
<213>  Artificial Sequence

<220>
<223>  RAG-344

<400>  92
ucuccaggag gacacagcat t                                                21


<210>  93
<211>  21
<212>  DNA
<213>  Artificial Sequence

<220>
<223>  RAG-345

<400>  93
gugcuguguc cuccuggagt t                                                21


<210>  94
<211>  21
<212>  DNA
<213>  Artificial Sequence

<220>
<223>  RAG-345

<400>  94
cuccaggagg acacagcact t                                                21


<210>  95
<211>  21
<212>  DNA
<213>  Artificial Sequence

<220>

<223>  RAG-346

<400>  95
agugcugugu ccuccuggat t                                                21


<210>  96
<211>  21
<212>  DNA
<213>  Artificial Sequence

<220>
<223>  RAG-346

<400>  96
uccaggagga cacagcacut t                                                21


<210>  97
<211>  21
<212>  DNA
<213>  Artificial Sequence

<220>
<223>  RAG-348

<400>  97
acagugcugu guccuccugt t                                                21


<210>  98
<211>  21
<212>  DNA
<213>  Artificial Sequence

<220>
<223>  RAG-348

<400>  98
caggaggaca cagcacugut t                                                21


<210>  99
<211>  21
<212>  DNA
<213>  Artificial Sequence

<220>
<223>  RAG-349

<400>  99
aacagugcug uguccuccut t                                                21


<210>  100
<211>  21
<212>  DNA
<213>  Artificial Sequence

<220>
<223>  RAG-349

<400>  100

aggaggacac agcacuguut t                                                21

<210>  101
<211>  21
<212>  DNA
<213>  Artificial Sequence

<220>
<223>  RAG-350

<400>  101
uaacagugcu guguccucct t                                                21

<210>  102
<211>  21
<212>  DNA
<213>  Artificial Sequence

<220>
<223>  RAG-350

<400>  102
ggaggacaca gcacuguuat t                                                21

<210>  103
<211>  21
<212>  DNA
<213>  Artificial Sequence

<220>
<223>  RAG-351

<400>  103
cuaacagugc uguguccuct t                                                21

<210>  104
<211>  21
<212>  DNA
<213>  Artificial Sequence

<220>
<223>  RAG-351

<400>  104
gaggacacag cacuguuagt t                                                21

<210>  105
<211>  21
<212>  DNA
<213>  Artificial Sequence

<220>
<223>  RAG-352

<400>  105
ucuaacagug cuguguccut t                                                21

<210>  106
<211>  21
<212>  DNA
<213>  Artificial Sequence

<220>
<223>  RAG-352

<400>  106
aggacacagc acuguuagat t                                                21


<210>  107
<211>  21
<212>  DNA
<213>  Artificial Sequence

<220>
<223>  RAG-423

<400>  107
ucuuuucagc ugcauugggt t                                                21


<210>  108
<211>  21
<212>  DNA
<213>  Artificial Sequence

<220>
<223>  RAG-423

<400>  108
cccaaugcag cugaaaagat t                                                21


<210>  109
<211>  21
<212>  DNA
<213>  Artificial Sequence

<220>
<223>  RAG-424

<400>  109
aucuuuucag cugcauuggt t                                                21


<210>  110
<211>  21
<212>  DNA
<213>  Artificial Sequence

<220>
<223>  RAG-424

<400>  110
ccaaugcagc ugaaaagaut t                                                21


<210>  111
<211>  21
<212>  DNA

<213>  Artificial Sequence

<220>
<223>  RAG-425

<400>  111
gaucuuuuca gcugcauugt t                                                21


<210>  112
<211>  21
<212>  DNA
<213>  Artificial Sequence

<220>
<223>  RAG-425

<400>  112
caaugcagcu gaaaagauct t                                                21


<210>  113
<211>  21
<212>  DNA
<213>  Artificial Sequence

<220>
<223>  RAG-426

<400>  113
ugaucuuuuc agcugcauut t                                                21


<210>  114
<211>  21
<212>  DNA
<213>  Artificial Sequence

<220>
<223>  RAG-426

<400>  114
aaugcagcug aaaagaucat t                                                21


<210>  115
<211>  21
<212>  DNA
<213>  Artificial Sequence

<220>
<223>  RAG-427

<400>  115
cugaucuuuu cagcugcaut t                                                21


<210>  116
<211>  21
<212>  DNA
<213>  Artificial Sequence

<220>

<223>  RAG-427

<400>  116
augcagcuga aaagaucagt t                                                21


<210>  117
<211>  21
<212>  DNA
<213>  Artificial Sequence

<220>
<223>  RAG-428

<400>  117
ccugaucuuu ucagcugcat t                                                21


<210>  118
<211>  21
<212>  DNA
<213>  Artificial Sequence

<220>
<223>  RAG-428

<400>  118
ugcagcugaa aagaucaggt t                                                21


<210>  119
<211>  21
<212>  DNA
<213>  Artificial Sequence

<220>
<223>  RAG-429

<400>  119
uccugaucuu uucagcugct t                                                21


<210>  120
<211>  21
<212>  DNA
<213>  Artificial Sequence

<220>
<223>  RAG-429

<400>  120
gcagcugaaa agaucaggat t                                                21


<210>  121
<211>  21
<212>  DNA
<213>  Artificial Sequence

<220>
<223>  RAG-430

<400>  121

cuccugaucu uuucagcugt t                                                21

<210>  122
<211>  21
<212>  DNA
<213>  Artificial Sequence

<220>
<223>  RAG-430

<400>  122
cagcugaaaa gaucaggagt t                                                21

<210>  123
<211>  21
<212>  DNA
<213>  Artificial Sequence

<220>
<223>  RAG-431

<400>  123
ccuccugauc uuuucagcut t                                                21

<210>  124
<211>  21
<212>  DNA
<213>  Artificial Sequence

<220>
<223>  RAG-431

<400>  124
agcugaaaag aucaggaggt t                                                21

<210>  125
<211>  21
<212>  DNA
<213>  Artificial Sequence

<220>
<223>  RAG-432

<400>  125
uccuccugau cuuuucagct t                                                21

<210>  126
<211>  21
<212>  DNA
<213>  Artificial Sequence

<220>
<223>  RAG-432

<400>  126
gcugaaaaga ucaggaggat t                                                21

<210>  127
<211>  21
<212>  DNA
<213>  Artificial Sequence

<220>
<223>  RAG-433

<400>  127
auccuccuga ucuuuucagt t                                                21


<210>  128
<211>  21
<212>  DNA
<213>  Artificial Sequence

<220>
<223>  RAG-433

<400>  128
cugaaaagau caggaggaut t                                                21


<210>  129
<211>  21
<212>  DNA
<213>  Artificial Sequence

<220>
<223>  RAG-434

<400>  129
cauccuccug aucuuuucat t                                                21


<210>  130
<211>  21
<212>  DNA
<213>  Artificial Sequence

<220>
<223>  RAG-434

<400>  130
ugaaaagauc aggaggaugt t                                                21


<210>  131
<211>  21
<212>  DNA
<213>  Artificial Sequence

<220>
<223>  RAG-435

<400>  131
ucauccuccu gaucuuuuct t                                                21


<210>  132
<211>  21
<212>  DNA

<213>  Artificial Sequence

<220>
<223>  RAG-435

<400>  132
gaaaagauca ggaggaugat t                                                21


<210>  133
<211>  21
<212>  DNA
<213>  Artificial Sequence

<220>
<223>  RAG-436

<400>  133
gucauccucc ugaucuuuut t                                                21


<210>  134
<211>  21
<212>  DNA
<213>  Artificial Sequence

<220>
<223>  RAG-436

<400>  134
aaaagaucag gaggaugact t                                                21


<210>  135
<211>  21
<212>  DNA
<213>  Artificial Sequence

<220>
<223>  RAG-437

<400>  135
ugucauccuc cugaucuuut t                                                21


<210>  136
<211>  21
<212>  DNA
<213>  Artificial Sequence

<220>
<223>  RAG-437

<400>  136
aaagaucagg aggaugacat t                                                21


<210>  137
<211>  21
<212>  DNA
<213>  Artificial Sequence

<220>

<223>  RAG-438

<400>  137
augucauccu ccugaucuut t                                                21


<210>  138
<211>  21
<212>  DNA
<213>  Artificial Sequence

<220>
<223>  RAG-438

<400>  138
aagaucagga ggaugacaut t                                                21


<210>  139
<211>  21
<212>  DNA
<213>  Artificial Sequence

<220>
<223>  RAG-439

<400>  139
aaugucaucc uccugaucut t                                                21


<210>  140
<211>  21
<212>  DNA
<213>  Artificial Sequence

<220>
<223>  RAG-439

<400>  140
agaucaggag gaugacauut t                                                21


<210>  141
<211>  21
<212>  DNA
<213>  Artificial Sequence

<220>
<223>  RAG-440

<400>  141
uaaugucauc cuccugauct t                                                21


<210>  142
<211>  21
<212>  DNA
<213>  Artificial Sequence

<220>
<223>  RAG-440

<400>  142

gaucaggagg augacauuat t                                                21

<210>  143
<211>  21
<212>  DNA
<213>  Artificial Sequence

<220>
<223>  RAG-441

<400>  143
uuaaugucau ccuccugaut t                                                21

<210>  144
<211>  21
<212>  DNA
<213>  Artificial Sequence

<220>
<223>  RAG-441

<400>  144
aucaggagga ugacauuaat t                                                21

<210>  145
<211>  21
<212>  DNA
<213>  Artificial Sequence

<220>
<223>  RAG-442

<400>  145
auuaauguca uccuccugat t                                                21

<210>  146
<211>  21
<212>  DNA
<213>  Artificial Sequence

<220>
<223>  RAG-442

<400>  146
ucaggaggau gacauuaaut t                                                21

<210>  147
<211>  21
<212>  DNA
<213>  Artificial Sequence

<220>
<223>  RAG-503

<400>  147
uggggcuuuu cuggaaauut t                                                21

<210>  148
<211>  21
<212>  DNA
<213>  Artificial Sequence

<220>
<223>  RAG-503

<400>  148
aauuuccaga aaagccccat t                                                21


<210>  149
<211>  21
<212>  DNA
<213>  Artificial Sequence

<220>
<223>  RAG-504

<400>  149
guggggcuuu ucuggaaaut t                                                21


<210>  150
<211>  21
<212>  DNA
<213>  Artificial Sequence

<220>
<223>  RAG-504

<400>  150
auuuccagaa aagccccact t                                                21


<210>  151
<211>  21
<212>  DNA
<213>  Artificial Sequence

<220>
<223>  RAG-505

<400>  151
   uguggggcuu uucuggaaat t                                                21


<210>  152
<211>  21
<212>  DNA
<213>  Artificial Sequence

<220>
<223>  RAG-505

<400>  152
uuuccagaaa agccccacat t                                                21


<210>  153
<211>  21
<212>  DNA

<213>  Artificial Sequence

<220>
<223>  RAG-506

<400>  153
uuguggggcu uuucuggaat t                                                21


<210>  154
<211>  21
<212>  DNA
<213>  Artificial Sequence

<220>
<223>  RAG-506

<400>  154
uuccagaaaa gccccacaat t                                                21


<210>  155
<211>  21
<212>  DNA
<213>  Artificial Sequence

<220>
<223>  RAG-507

<400>  155
auuguggggc uuuucuggat t                                                21


<210>  156
<211>  21
<212>  DNA
<213>  Artificial Sequence

<220>
<223>  RAG-507

<400>  156
uccagaaaag ccccacaaut t                                                21


<210>  157
<211>  21
<212>  DNA
<213>  Artificial Sequence

<220>
<223>  RAG-508

<400>  157
uauugugggg cuuuucuggt t                                                21


<210>  158
<211>  21
<212>  DNA
<213>  Artificial Sequence

<220>

<223>  RAG-508

<400>  158
ccagaaaagc cccacaauat t                                                21


<210>  159
<211>  21
<212>  DNA
<213>  Artificial Sequence

<220>
<223>  RAG-509

<400>  159
auauuguggg gcuuuucugt t                                                21


<210>  160
<211>  21
<212>  DNA
<213>  Artificial Sequence

<220>
<223>  RAG-509

<400>  160
cagaaaagcc ccacaauaut t                                                21


<210>  161
<211>  21
<212>  DNA
<213>  Artificial Sequence

<220>
<223>  RAG-510

<400>  161
gauauugugg ggcuuuucut t                                                21


<210>  162
<211>  21
<212>  DNA
<213>  Artificial Sequence

<220>
<223>  RAG-510

<400>  162
agaaaagccc cacaauauct t                                                21


<210>  163
<211>  21
<212>  DNA
<213>  Artificial Sequence

<220>
<223>  RAG-511

<400>  163

ugauauugug gggcuuuuct t                                                21

<210>  164
<211>  21
<212>  DNA
<213>  Artificial Sequence

<220>
<223>  RAG-511

<400>  164
gaaaagcccc acaauaucat t                                                21

<210>  165
<211>  21
<212>  DNA
<213>  Artificial Sequence

<220>
<223>  RAG-512

<400>  165
gugauauugu ggggcuuuut t                                                21

<210>  166
<211>  21
<212>  DNA
<213>  Artificial Sequence

<220>
<223>  RAG-512

<400>  166
aaaagcccca caauaucact t                                                21

<210>  167
<211>  21
<212>  DNA
<213>  Artificial Sequence

<220>
<223>  RAG-513

<400>  167
ggugauauug uggggcuuut t                                                21

<210>  168
<211>  21
<212>  DNA
<213>  Artificial Sequence

<220>
<223>  RAG-513

<400>  168
aaagccccac aauaucacct t                                                21

<210>  169
<211>  21
<212>  DNA
<213>  Artificial Sequence

<220>
<223>  RAG-514

<400>  169
aggugauauu guggggcuut t                                                21


<210>  170
<211>  21
<212>  DNA
<213>  Artificial Sequence

<220>
<223>  RAG-514

<400>  170
aagccccaca auaucaccut t                                                21


<210>  171
<211>  21
<212>  DNA
<213>  Artificial Sequence

<220>
<223>  RAG-522

<400>  171
gggaauagag gugauauugt t                                                21


<210>  172
<211>  21
<212>  DNA
<213>  Artificial Sequence

<220>
<223>  RAG-522

<400>  172
caauaucacc ucuauuccct t                                                21


<210>  173
<211>  21
<212>  DNA
<213>  Artificial Sequence

<220>
<223>  RAG-523

<400>  173
ugggaauaga ggugauauut t                                                21


<210>  174
<211>  21
<212>  DNA

<213>  Artificial Sequence

<220>
<223>  RAG-523

<400>  174
aauaucaccu cuauucccat t                                                21


<210>  175
<211>  21
<212>  DNA
<213>  Artificial Sequence

<220>
<223>  RAG-524

<400>  175
gugggaauag aggugauaut t                                                21


<210>  176
<211>  21
<212>  DNA
<213>  Artificial Sequence

<220>
<223>  RAG-524

<400>  176
auaucaccuc uauucccact t                                                21


<210>  177
<211>  21
<212>  DNA
<213>  Artificial Sequence

<220>
<223>  RAG-525

<400>  177
agugggaaua gaggugauat t                                                21


<210>  178
<211>  21
<212>  DNA
<213>  Artificial Sequence

<220>
<223>  RAG-525

<400>  178
uaucaccucu auucccacut t                                                21


<210>  179
<211>  21
<212>  DNA
<213>  Artificial Sequence

<220>

<223>  RAG-526

<400>  179
cagugggaau agaggugaut t                                                21


<210>  180
<211>  21
<212>  DNA
<213>  Artificial Sequence

<220>
<223>  RAG-526

<400>  180
aucaccucua uucccacugt t                                                21


<210>  181
<211>  21
<212>  DNA
<213>  Artificial Sequence

<220>
<223>  RAG-527

<400>  181
ucagugggaa uagaggugat t                                                21


<210>  182
<211>  21
<212>  DNA
<213>  Artificial Sequence

<220>
<223>  RAG-527

<400>  182
ucaccucuau ucccacugat t                                                21


<210>  183
<211>  21
<212>  DNA
<213>  Artificial Sequence

<220>
<223>  RAG-528

<400>  183
aucaguggga auagaggugt t                                                21


<210>  184
<211>  21
<212>  DNA
<213>  Artificial Sequence

<220>
<223>  RAG-528

<400>  184

caccucuauu cccacugaut t                                                21

<210>  185
<211>  21
<212>  DNA
<213>  Artificial Sequence

<220>
<223>  RAG-529

<400>  185
gaucaguggg aauagaggut t                                                21

<210>  186
<211>  21
<212>  DNA
<213>  Artificial Sequence

<220>
<223>  RAG-529

<400>  186
accucuauuc ccacugauct t                                                21

<210>  187
<211>  21
<212>  DNA
<213>  Artificial Sequence

<220>
<223>  RAG-530

<400>  187
ggaucagugg gaauagaggt t                                                21

<210>  188
<211>  21
<212>  DNA
<213>  Artificial Sequence

<220>
<223>  RAG-530

<400>  188
ccucuauucc cacugaucct t                                                21

<210>  189
<211>  21
<212>  DNA
<213>  Artificial Sequence

<220>
<223>  RAG-531

<400>  189
gggaucagug ggaauagagt t                                                21

<210>  190
<211>  21
<212>  DNA
<213>  Artificial Sequence

<220>
<223>  RAG-531

<400>  190
cucuauuccc acugauccct t                                                21


<210>  191
<211>  21
<212>  DNA
<213>  Artificial Sequence

<220>
<223>  RAG-532

<400>  191
agggaucagu gggaauagat t                                                21


<210>  192
<211>  21
<212>  DNA
<213>  Artificial Sequence

<220>
<223>  RAG-532

<400>  192
ucuauuccca cugaucccut t                                                21


<210>  193
<211>  21
<212>  DNA
<213>  Artificial Sequence

<220>
<223>  RAG-533

<400>  193
gagggaucag ugggaauagt t                                                21


<210>  194
<211>  21
<212>  DNA
<213>  Artificial Sequence

<220>
<223>  RAG-533

<400>  194
cuauucccac ugaucccuct t                                                21


<210>  195
<211>  21
<212>  DNA

<213>  Artificial Sequence

<220>
<223>  RAG-534

<400>  195
ugagggauca gugggaauat t                                                21


<210>  196
<211>  21
<212>  DNA
<213>  Artificial Sequence

<220>
<223>  RAG-534

<400>  196
uauucccacu gaucccucat t                                                21


<210>  197
<211>  21
<212>  DNA
<213>  Artificial Sequence

<220>
<223>  RAG-535

<400>  197
gugagggauc agugggaaut t                                                21


<210>  198
<211>  21
<212>  DNA
<213>  Artificial Sequence

<220>
<223>  RAG-535

<400>  198
auucccacug aucccucact t                                                21


<210>  199
<211>  21
<212>  DNA
<213>  Artificial Sequence

<220>
<223>  RAG-536

<400>  199
agugagggau cagugggaat t                                                21


<210>  200
<211>  21
<212>  DNA
<213>  Artificial Sequence

<220>

<223>  RAG-536

<400>  200
uucccacuga ucccucacut t                                                21


<210>  201
<211>  21
<212>  DNA
<213>  Artificial Sequence

<220>
<223>  RAG-537

<400>  201
uagugaggga ucagugggat t                                                21


<210>  202
<211>  21
<212>  DNA
<213>  Artificial Sequence

<220>
<223>  RAG-537

<400>  202
ucccacugau cccucacuat t                                                21


<210>  203
<211>  21
<212>  DNA
<213>  Artificial Sequence

<220>
<223>  RAG-538

<400>  203
cuagugaggg aucagugggt t                                                21


<210>  204
<211>  21
<212>  DNA
<213>  Artificial Sequence

<220>
<223>  RAG-538

<400>  204
cccacugauc ccucacuagt t                                                21


<210>  205
<211>  21
<212>  DNA
<213>  Artificial Sequence

<220>
<223>  RAG-540

<400>  205

accuagugag ggaucagugt t                                              21


<210>  206
<211>  21
<212>  DNA
<213>  Artificial Sequence

<220>
<223>  RAG-540

<400>  206
cacugauccc ucacuaggut t                                              21


<210>  207
<211>  21
<212>  DNA
<213>  Artificial Sequence

<220>
<223>  RAG-541

<400>  207
gaccuaguga gggaucagut t                                              21


<210>  208
<211>  21
<212>  DNA
<213>  Artificial Sequence

<220>
<223>  RAG-541

<400>  208
acugaucccu cacuagguct t                                              21


<210>  209
<211>  21
<212>  DNA
<213>  Artificial Sequence

<220>
<223>  RAG-542

<400>  209
ugaccuagug agggaucagt t                                              21


<210>  210
<211>  21
<212>  DNA
<213>  Artificial Sequence

<220>
<223>  RAG-542

<400>  210
cugaucccuc acuaggucat t                                              21

<210>  211
<211>  21
<212>  DNA
<213>  Artificial Sequence

<220>
<223>  RAG-543

<400>  211
gugaccuagu gagggaucat t                                                21


<210>  212
<211>  21
<212>  DNA
<213>  Artificial Sequence

<220>
<223>  RAG-543

<400>  212
ugaucccuca cuaggucact t                                                21


<210>  213
<211>  21
<212>  DNA
<213>  Artificial Sequence

<220>
<223>  RAG-544

<400>  213
ggugaccuag ugagggauct t                                                21


<210>  214
<211>  21
<212>  DNA
<213>  Artificial Sequence

<220>
<223>  RAG-544

<400>  214
gaucccucac uaggucacct t                                                21


<210>  215
<211>  21
<212>  DNA
<213>  Artificial Sequence

<220>
<223>  RAG-545

<400>  215
aggugaccua gugagggaut t                                                21


<210>  216
<211>  21
<212>  DNA

<213>  Artificial Sequence

<220>
<223>  RAG-545

<400>  216
aucccucacu aggucaccut t                                                21


<210>  217
<211>  21
<212>  DNA
<213>  Artificial Sequence

<220>
<223>  RAG-546

<400>  217
gaggugaccu agugagggat t                                                21


<210>  218
<211>  21
<212>  DNA
<213>  Artificial Sequence

<220>
<223>  RAG-546

<400>  218
ucccucacua ggucaccuct t                                                21


<210>  219
<211>  21
<212>  DNA
<213>  Artificial Sequence

<220>
<223>  RAG-549

<400>  219
ggagagguga ccuagugagt t                                                21


<210>  220
<211>  21
<212>  DNA
<213>  Artificial Sequence

<220>
<223>  RAG-549

<400>  220
cucacuaggu caccucucct t                                                21


<210>  221
<211>  21
<212>  DNA
<213>  Artificial Sequence

<220>

<223>  RAG-550

<400>  221
gggagaggug accuagugat t                                                21


<210>  222
<211>  21
<212>  DNA
<213>  Artificial Sequence

<220>
<223>  RAG-550

<400>  222
ucacuagguc accucuccct t                                                21


<210>  223
<211>  21
<212>  DNA
<213>  Artificial Sequence

<220>
<223>  RAG-551

<400>  223
ugggagaggu gaccuagugt t                                                21


<210>  224
<211>  21
<212>  DNA
<213>  Artificial Sequence

<220>
<223>  RAG-551

<400>  224
cacuagguca ccucucccat t                                                21


<210>  225
<211>  21
<212>  DNA
<213>  Artificial Sequence

<220>
<223>  RAG-552

<400>  225
cugggagagg ugaccuagut t                                                21


<210>  226
<211>  21
<212>  DNA
<213>  Artificial Sequence

<220>
<223>  RAG-552

<400>  226

acuaggucac cucucccagt t                                                21

<210>  227
<211>  21
<212>  DNA
<213>  Artificial Sequence

<220>
<223>  RAG-553

<400>  227
ucugggagag gugaccuagt t                                                21

<210>  228
<211>  21
<212>  DNA
<213>  Artificial Sequence

<220>
<223>  RAG-553

<400>  228
cuaggucacc ucucccagat t                                                21

<210>  229
<211>  21
<212>  DNA
<213>  Artificial Sequence

<220>
<223>  RAG-554

<400>  229
uucugggaga ggugaccuat t                                                21

<210>  230
<211>  21
<212>  DNA
<213>  Artificial Sequence

<220>
<223>  RAG-554

<400>  230
uaggucaccu cucccagaat t                                                21

<210>  231
<211>  21
<212>  DNA
<213>  Artificial Sequence

<220>
<223>  RAG-569

<400>  231
aggugcucca ggugcuucut t                                                21

<210>  232
<211>  21
<212>  DNA
<213>  Artificial Sequence

<220>
<223>  RAG-569

<400>  232
agaagcaccu ggagcaccut t                                                21


<210>  233
<211>  21
<212>  DNA
<213>  Artificial Sequence

<220>
<223>  RAG-570

<400>  233
uaggugcucc aggugcuuct t                                                21


<210>  234
<211>  21
<212>  DNA
<213>  Artificial Sequence

<220>
<223>  RAG-570

<400>  234
gaagcaccug gagcaccuat t                                                21


<210>  235
<211>  21
<212>  DNA
<213>  Artificial Sequence

<220>
<223>  RAG-574

<400>  235
ugucuaggug cuccaggugt t                                                21


<210>  236
<211>  21
<212>  DNA
<213>  Artificial Sequence

<220>
<223>  RAG-574

<400>  236
caccuggagc accuagacat t                                                21


<210>  237
<211>  21
<212>  DNA

<213>  Artificial Sequence

<220>
<223>  RAG-576

<400>  237
ggugucuagg ugcuccaggt t                                                21


<210>  238
<211>  21
<212>  DNA
<213>  Artificial Sequence

<220>
<223>  RAG-576

<400>  238
ccuggagcac cuagacacct t                                                21


<210>  239
<211>  21
<212>  DNA
<213>  Artificial Sequence

<220>
<223>  RAG-577

<400>  239
gggugucuag gugcuccagt t                                                21


<210>  240
<211>  21
<212>  DNA
<213>  Artificial Sequence

<220>
<223>  RAG-577

<400>  240
cuggagcacc uagacaccct t                                                21


<210>  241
<211>  21
<212>  DNA
<213>  Artificial Sequence

<220>
<223>  RAG-578

<400>  241
ggggugucua ggugcuccat t                                                21


<210>  242
<211>  21
<212>  DNA
<213>  Artificial Sequence

<220>

<223>  RAG-578

<400>  242
uggagcaccu agacacccct t                                                21


<210>  243
<211>  21
<212>  DNA
<213>  Artificial Sequence

<220>
<223>  RAG-579

<400>  243
uggggugucu aggugcucct t                                                21


<210>  244
<211>  21
<212>  DNA
<213>  Artificial Sequence

<220>
<223>  RAG-579

<400>  244
ggagcaccua gacaccccat t                                                21


<210>  245
<211>  21
<212>  DNA
<213>  Artificial Sequence

<220>
<223>  RAG-580

<400>  245
   uuggggugug uaggugcuct t                                               21


<210>  246
<211>  21
<212>  DNA
<213>  Artificial Sequence

<220>
<223>  RAG-580

<400>  246
gagcaccuag acaccccaat t                                                21


<210>  247
<211>  21
<212>  DNA
<213>  Artificial Sequence

<220>
<223>  RAG-583

<400>  247

uuguuggggu gucuaggugt t                                                21

<210>  248
<211>  21
<212>  DNA
<213>  Artificial Sequence

<220>
<223>  RAG-583

<400>  248
caccuagaca ccccaacaat t                                                21

<210>  249
<211>  21
<212>  DNA
<213>  Artificial Sequence

<220>
<223>  RAG-584

<400>  249
uuuguugggg ugucuaggut t                                                21

<210>  250
<211>  21
<212>  DNA
<213>  Artificial Sequence

<220>
<223>  RAG-584

<400>  250
accuagacac cccaacaaat t                                                21

<210>  251
<211>  21
<212>  DNA
<213>  Artificial Sequence

<220>
<223>  RAG-585

<400>  251
cuuuguuggg gugucuaggt t                                                21

<210>  252
<211>  21
<212>  DNA
<213>  Artificial Sequence

<220>
<223>  RAG-585

<400>  252
ccuagacacc ccaacaaagt t                                                21

<210>  253
<211>  21
<212>  DNA
<213>  Artificial Sequence

<220>
<223>  RAG-650

<400>  253
aguggaccuc aauuuccuct t                                                21


<210>  254
<211>  21
<212>  DNA
<213>  Artificial Sequence

<220>
<223>  RAG-650

<400>  254
gaggaaauug agguccacut t                                                21


<210>  255
<211>  21
<212>  DNA
<213>  Artificial Sequence

<220>
<223>  RAG-651

<400>  255
caguggaccu caauuuccut t                                                21


<210>  256
<211>  21
<212>  DNA
<213>  Artificial Sequence

<220>
<223>  RAG-651

<400>  256
aggaaauuga gguccacugt t                                                21


<210>  257
<211>  21
<212>  DNA
<213>  Artificial Sequence

<220>
<223>  RAG-652

<400>  257
ucaguggacc ucaauuucct t                                                21


<210>  258
<211>  21
<212>  DNA

<213>  Artificial Sequence

<220>
<223>  RAG-652

<400>  258
ggaaauugag guccacugat t                                                21


<210>  259
<211>  21
<212>  DNA
<213>  Artificial Sequence

<220>
<223>  RAG-653

<400>  259
uucaguggac cucaauuuct t                                                21


<210>  260
<211>  21
<212>  DNA
<213>  Artificial Sequence

<220>
<223>  RAG-653

<400>  260
gaaauugagg uccacugaat t                                                21


<210>  261
<211>  21
<212>  DNA
<213>  Artificial Sequence

<220>
<223>  RAG-654

<400>  261
guucagugga ccucaauuut t                                                21


<210>  262
<211>  21
<212>  DNA
<213>  Artificial Sequence

<220>
<223>  RAG-654

<400>  262
aaauugaggu ccacugaact t                                                21


<210>  263
<211>  21
<212>  DNA
<213>  Artificial Sequence

<220>

<223>  RAG-655

<400>  263
aguucagugg accucaauut t                                                21


<210>  264
<211>  21
<212>  DNA
<213>  Artificial Sequence

<220>
<223>  RAG-655

<400>  264
aauugagguc cacugaacut t                                                21


<210>  265
<211>  21
<212>  DNA
<213>  Artificial Sequence

<220>
<223>  RAG-656

<400>  265
aaguucagug gaccucaaut t                                                21


<210>  266
<211>  21
<212>  DNA
<213>  Artificial Sequence

<220>
<223>  RAG-656

<400>  266
auugaggucc acugaacuut t                                                21


<210>  267
<211>  21
<212>  DNA
<213>  Artificial Sequence

<220>
<223>  RAG-657

<400>  267
uaaguucagu ggaccucaat t                                                21


<210>  268
<211>  21
<212>  DNA
<213>  Artificial Sequence

<220>
<223>  RAG-657

<400>  268

uugaggucca cugaacuuat t                                                21

<210>  269
<211>  21
<212>  DNA
<213>  Artificial Sequence

<220>
<223>  RAG-658

<400>  269
uuaaguucag uggaccucat t                                                21

<210>  270
<211>  21
<212>  DNA
<213>  Artificial Sequence

<220>
<223>  RAG-658

<400>  270
ugagguccac ugaacuuaat t                                                21

<210>  271
<211>  21
<212>  DNA
<213>  Artificial Sequence

<220>
<223>  RAG-659

<400>  271
cuuaaguuca guggaccuct t                                                21

<210>  272
<211>  21
<212>  DNA
<213>  Artificial Sequence

<220>
<223>  RAG-659

<400>  272
gagguccacu gaacuuaagt t                                                21

<210>  273
<211>  21
<212>  DNA
<213>  Artificial Sequence

<220>
<223>  RAG-660

<400>  273
acuuaaguuc aguggaccut t                                                21

<210>  274
<211>  21
<212>  DNA
<213>  Artificial Sequence

<220>
<223>  RAG-660

<400>  274
agguccacug aacuuaagut t                                                21


<210>  275
<211>  21
<212>  DNA
<213>  Artificial Sequence

<220>
<223>  RAG-661

<400>  275
uacuuaaguu caguggacct t                                                21


<210>  276
<211>  21
<212>  DNA
<213>  Artificial Sequence

<220>
<223>  RAG-661

<400>  276
gguccacuga acuuaaguat t                                                21


<210>  277
<211>  21
<212>  DNA
<213>  Artificial Sequence

<220>
<223>  RAG-662

<400>  277
auacuuaagu ucaguggact t                                                21


<210>  278
<211>  21
<212>  DNA
<213>  Artificial Sequence

<220>
<223>  RAG-662

<400>  278
guccacugaa cuuaaguaut t                                                21


<210>  279
<211>  21
<212>  DNA

<213>  Artificial Sequence

<220>
<223>  RAG-682

<400>  279
gugacaauca acaacuuugt t                                                21


<210>  280
<211>  21
<212>  DNA
<213>  Artificial Sequence

<220>
<223>  RAG-682

<400>  280
caaaguuguu gauugucact t                                                21


<210>  281
<211>  21
<212>  DNA
<213>  Artificial Sequence

<220>
<223>  RAG-685

<400>  281
caugugacaa ucaacaacut t                                                21


<210>  282
<211>  21
<212>  DNA
<213>  Artificial Sequence

<220>
<223>  RAG-685

<400>  282
aguuguugau ugucacaugt t                                                21


<210>  283
<211>  21
<212>  DNA
<213>  Artificial Sequence

<220>
<223>  RAG-686

<400>  283
gcaugugaca aucaacaact t                                                21


<210>  284
<211>  21
<212>  DNA
<213>  Artificial Sequence

<220>

<223>  RAG-686

<400>  284
guuguugauu gucacaugct t                                                21


<210>  285
<211>  21
<212>  DNA
<213>  Artificial Sequence

<220>
<223>  RAG-687

<400>  285
agcaugugac aaucaacaat t                                                21


<210>  286
<211>  21
<212>  DNA
<213>  Artificial Sequence

<220>
<223>  RAG-687

<400>  286
uuguugauug ucacaugcut t                                                21


<210>  287
<211>  21
<212>  DNA
<213>  Artificial Sequence

<220>
<223>  RAG-688

<400>  287
aagcauguga caaucaacat t                                                21


<210>  288
<211>  21
<212>  DNA
<213>  Artificial Sequence

<220>
<223>  RAG-688

<400>  288
uguugauugu cacaugcuut t                                                21


<210>  289
<211>  21
<212>  DNA
<213>  Artificial Sequence

<220>
<223>  RAG-689

<400>  289

gaagcaugug acaaucaact t                                                21

<210>  290
<211>  21
<212>  DNA
<213>  Artificial Sequence

<220>
<223>  RAG-689

<400>  290
guugauuguc acaugcuuct t                                                21

<210>  291
<211>  21
<212>  DNA
<213>  Artificial Sequence

<220>
<223>  RAG-690

<400>  291
ggaagcaugu gacaaucaat t                                                21

<210>  292
<211>  21
<212>  DNA
<213>  Artificial Sequence

<220>
<223>  RAG-690

<400>  292
uugauuguca caugcuucct t                                                21

<210>  293
<211>  21
<212>  DNA
<213>  Artificial Sequence

<220>
<223>  RAG-691

<400>  293
cggaagcaug ugacaaucat t                                                21

<210>  294
<211>  21
<212>  DNA
<213>  Artificial Sequence

<220>
<223>  RAG-691

<400>  294
ugauugucac augcuuccgt t                                                21

<210>  295
<211>  21
<212>  DNA
<213>  Artificial Sequence

<220>
<223>  RAG-692

<400>  295
ccggaagcau gugacaauct t                                                21


<210>  296
<211>  21
<212>  DNA
<213>  Artificial Sequence

<220>
<223>  RAG-692

<400>  296
gauugucaca ugcuuccggt t                                                21


<210>  297
<211>  21
<212>  DNA
<213>  Artificial Sequence

<220>
<223>  RAG-693

<400>  297
cccggaagca ugugacaaut t                                                21


<210>  298
<211>  21
<212>  DNA
<213>  Artificial Sequence

<220>
<223>  RAG-693

<400>  298
auugucacau gcuuccgggt t                                                21


<210>  299
<211>  21
<212>  DNA
<213>  Artificial Sequence

<220>
<223>  RAG-694

<400>  299
ucccggaagc augugacaat t                                                21


<210>  300
<211>  21
<212>  DNA

<213>  Artificial Sequence

<220>
<223>  RAG-694

<400>  300
uugucacaug cuucccggat t                                                21


<210>  301
<211>  21
<212>  DNA
<213>  Artificial Sequence

<220>
<223>  RAG-695

<400>  301
uucccggaag caugugacat t                                                21


<210>  302
<211>  21
<212>  DNA
<213>  Artificial Sequence

<220>
<223>  RAG-695

<400>  302
ugucacaugc uuccgggaat t                                                21


<210>  303
<211>  21
<212>  DNA
<213>  Artificial Sequence

<220>
<223>  RAG-696

<400>  303
cuucccggaa gcaugugact t                                                21


<210>  304
<211>  21
<212>  DNA
<213>  Artificial Sequence

<220>
<223>  RAG-696

<400>  304
gucacaugcu uccgggaagt t                                                21


<210>  305
<211>  21
<212>  DNA
<213>  Artificial Sequence

<220>

<223>  RAG-697

<400>  305
ccuucccgga agcaugugat t                                                21


<210>  306
<211>  21
<212>  DNA
<213>  Artificial Sequence

<220>
<223>  RAG-697

<400>  306
ucacaugcuu ccgggaaggt t                                                21


<210>  307
<211>  21
<212>  DNA
<213>  Artificial Sequence

<220>
<223>  RAG-698

<400>  307
uccuucccgg aagcaugugt t                                                21


<210>  308
<211>  21
<212>  DNA
<213>  Artificial Sequence

<220>
<223>  RAG-698

<400>  308
cacaugcuuc cgggaaggat t                                                21


<210>  309
<211>  21
<212>  DNA
<213>  Artificial Sequence

<220>
<223>  RAG-699

<400>  309
cuccuucccg gaagcaugut t                                                21


<210>  310
<211>  21
<212>  DNA
<213>  Artificial Sequence

<220>
<223>  RAG-699

<400>  310

acaugcuucc gggaaggagt t                                                21

<210>  311
<211>  21
<212>  DNA
<213>  Artificial Sequence

<220>
<223>  RAG-700

<400>  311
ccuccuuccc ggaagcaugt t                                                21

<210>  312
<211>  21
<212>  DNA
<213>  Artificial Sequence

<220>
<223>  RAG-700

<400>  312
caugcuuccg ggaaggaggt t                                                21

<210>  313
<211>  21
<212>  DNA
<213>  Artificial Sequence

<220>
<223>  RAG-701

<400>  313
cccuccuucc cggaagcaut t                                                21

<210>  314
<211>  21
<212>  DNA
<213>  Artificial Sequence

<220>
<223>  RAG-701

<400>  314
augcuuccgg gaaggagggt t                                                21

<210>  315
<211>  21
<212>  DNA
<213>  Artificial Sequence

<220>
<223>  RAG-702

<400>  315
ucccuccuuc ccggaagcat t                                                21

<210>  316
<211>  21
<212>  DNA
<213>  Artificial Sequence

<220>
<223>  RAG-702

<400>  316
ugcuuccggg aaggagggat t                                                21


<210>  317
<211>  21
<212>  DNA
<213>  Artificial Sequence

<220>
<223>  RAG-704

<400>  317
auucccuccu ucccggaagt t                                                21


<210>  318
<211>  21
<212>  DNA
<213>  Artificial Sequence

<220>
<223>  RAG-704

<400>  318
cuuccgggaa ggagggaaut t                                                21


<210>  319
<211>  21
<212>  DNA
<213>  Artificial Sequence

<220>
<223>  RAG-705

<400>  319
aauucccucc uucccggaat t                                                21


<210>  320
<211>  21
<212>  DNA
<213>  Artificial Sequence

<220>
<223>  RAG-705

<400>  320
uuccgggaag gagggaauut t                                                21


<210>  321
<211>  21
<212>  DNA

<213>  Artificial Sequence

<220>
<223>  RAG-710

<400>  321
ucuccaauuc ccuccuucct t                                                21


<210>  322
<211>  21
<212>  DNA
<213>  Artificial Sequence

<220>
<223>  RAG-710

<400>  322
ggaaggaggg aauuggagat t                                                21


<210>  323
<211>  21
<212>  DNA
<213>  Artificial Sequence

<220>
<223>  RAG-711

<400>  323
cucuccaauu cccuccuuct t                                                21


<210>  324
<211>  21
<212>  DNA
<213>  Artificial Sequence

<220>
<223>  RAG-711

<400>  324
gaaggaggga auuggagagt t                                                21


<210>  325
<211>  21
<212>  DNA
<213>  Artificial Sequence

<220>
<223>  RAG-712

<400>  325
ucucuccaau ucccuccuut t                                                21


<210>  326
<211>  21
<212>  DNA
<213>  Artificial Sequence

<220>

<223>  RAG-712

<400>  326
aaggagggaa uuggagagat t                                                21


<210>  327
<211>  21
<212>  DNA
<213>  Artificial Sequence

<220>
<223>  RAG-713

<400>  327
gucucuccaa uucccuccut t                                                21


<210>  328
<211>  21
<212>  DNA
<213>  Artificial Sequence

<220>
<223>  RAG-713

<400>  328
aggagggaau uggagagact t                                                21


<210>  329
<211>  21
<212>  DNA
<213>  Artificial Sequence

<220>
<223>  RAG-714

<400>  329
agucucucca auucccucct t                                                21


<210>  330
<211>  21
<212>  DNA
<213>  Artificial Sequence

<220>
<223>  RAG-714

<400>  330
ggagggaauu ggagagacut t                                                21


<210>  331
<211>  21
<212>  DNA
<213>  Artificial Sequence

<220>
<223>  RAG-715

<400>  331

uagucucucc aauucccuct t                                                21

<210>  332
<211>  21
<212>  DNA
<213>  Artificial Sequence

<220>
<223>  RAG-715

<400>  332
gagggaauug gagagacuat t                                                21

<210>  333
<211>  21
<212>  DNA
<213>  Artificial Sequence

<220>
<223>  RAG-716

<400>  333
guagucucuc caauucccut t                                                21

<210>  334
<211>  21
<212>  DNA
<213>  Artificial Sequence

<220>
<223>  RAG-716

<400>  334
agggaauugg agagacuact t                                                21

<210>  335
<211>  21
<212>  DNA
<213>  Artificial Sequence

<220>
<223>  RAG-717

<400>  335
gguagucucu ccaauuccct t                                                21

<210>  336
<211>  21
<212>  DNA
<213>  Artificial Sequence

<220>
<223>  RAG-717

<400>  336
gggaauugga gagacuacct t                                                21

<210>  337
<211>  21
<212>  DNA
<213>  Artificial Sequence

<220>
<223>  RAG-820

<400>  337
ugcaaccaca gggauuucut t                                                21


<210>  338
<211>  21
<212>  DNA
<213>  Artificial Sequence

<220>
<223>  RAG-820

<400>  338
agaaaucccu gugguugcat t                                                21


<210>  339
<211>  21
<212>  DNA
<213>  Artificial Sequence

<220>
<223>  RAG-821

<400>  339
cugcaaccac agggauuuct t                                                21


<210>  340
<211>  21
<212>  DNA
<213>  Artificial Sequence

<220>
<223>  RAG-821

<400>  340
gaaaucccug ugguugcagt t                                                21


<210>  341
<211>  21
<212>  DNA
<213>  Artificial Sequence

<220>
<223>  RAG-822

<400>  341
gcugcaacca cagggauuut t                                                21


<210>  342
<211>  21
<212>  DNA

<213>  Artificial Sequence

<220>
<223>  RAG-822

<400>  342
aaaucccugu gguugcagct t                                                21


<210>  343
<211>  21
<212>  DNA
<213>  Artificial Sequence

<220>
<223>  RAG-823

<400>  343
ugcugcaacc acagggauut t                                                21


<210>  344
<211>  21
<212>  DNA
<213>  Artificial Sequence

<220>
<223>  RAG-823

<400>  344
aaucccugug guugcagcat t                                                21


<210>  345
<211>  21
<212>  DNA
<213>  Artificial Sequence

<220>
<223>  RAG-824

<400>  345
cugcugcaac cacagggaut t                                                21


<210>  346
<211>  21
<212>  DNA
<213>  Artificial Sequence

<220>
<223>  RAG-824

<400>  346
aucccugugg uugcagcagt t                                                21


<210>  347
<211>  21
<212>  DNA
<213>  Artificial Sequence

<220>

<223>  RAG-825

<400>  347
gcugcugcaa ccacagggat t                                                21


<210>  348
<211>  21
<212>  DNA
<213>  Artificial Sequence

<220>
<223>  RAG-825

<400>  348
ucccuguggu ugcagcagct t                                                21


<210>  349
<211>  21
<212>  DNA
<213>  Artificial Sequence

<220>
<223>  RAG-826

<400>  349
agcugcugca accacagggt t                                                21


<210>  350
<211>  21
<212>  DNA
<213>  Artificial Sequence

<220>
<223>  RAG-826

<400>  350
cccugugguu gcagcagcut t                                                21


<210>  351
<211>  21
<212>  DNA
<213>  Artificial Sequence

<220>
<223>  RAG-828

<400>  351
aaagcugcug caaccacagt t                                                21


<210>  352
<211>  21
<212>  DNA
<213>  Artificial Sequence

<220>
<223>  RAG-828

<400>  352

cugugguugc agcagcuuut t                                                21

<210>  353
<211>  21
<212>  DNA
<213>  Artificial Sequence

<220>
<223>  RAG-829

<400>  353
caaagcugcu gcaaccacat t                                                21

<210>  354
<211>  21
<212>  DNA
<213>  Artificial Sequence

<220>
<223>  RAG-829

<400>  354
ugugguugca gcagcuuugt t                                                21

<210>  355
<211>  21
<212>  DNA
<213>  Artificial Sequence

<220>
<223>  RAG-830

<400>  355
acaaagcugc ugcaaccact t                                                21

<210>  356
<211>  21
<212>  DNA
<213>  Artificial Sequence

<220>
<223>  RAG-830

<400>  356
gugguugcag cagcuuugut t                                                21

<210>  357
<211>  21
<212>  DNA
<213>  Artificial Sequence

<220>
<223>  RAG-831

<400>  357
aacaaagcug cugcaaccat t                                                21

<210>  358
<211>  21
<212>  DNA
<213>  Artificial Sequence

<220>
<223>  RAG-831

<400>  358
ugguugcagc agcuuuguut t                                                21


<210>  359
<211>  21
<212>  DNA
<213>  Artificial Sequence

<220>
<223>  RAG-832

<400>  359
caacaaagcu gcugcaacct t                                                21


<210>  360
<211>  21
<212>  DNA
<213>  Artificial Sequence

<220>
<223>  RAG-832

<400>  360
gguugcagca gcuuuguugt t                                                21


<210>  361
<211>  21
<212>  DNA
<213>  Artificial Sequence

<220>
<223>  RAG-833

<400>  361
ccaacaaagc ugcugcaact t                                                21


<210>  362
<211>  21
<212>  DNA
<213>  Artificial Sequence

<220>
<223>  RAG-833

<400>  362
guugcagcag cuuuguuggt t                                                21


<210>  363
<211>  21
<212>  DNA

<213>  Artificial Sequence

<220>
<223>  RAG-834

<400>  363
gccaacaaag cugcugcaat t                                                21


<210>  364
<211>  21
<212>  DNA
<213>  Artificial Sequence

<220>
<223>  RAG-834

<400>  364
uugcagcagc uuuguuggct t                                                21


<210>  365
<211>  21
<212>  DNA
<213>  Artificial Sequence

<220>
<223>  RAG-835

<400>  365
ggccaacaaa gcugcugcat t                                                21


<210>  366
<211>  21
<212>  DNA
<213>  Artificial Sequence

<220>
<223>  RAG-835

<400>  366
ugcagcagcu uuguuggcct t                                                21


<210>  367
<211>  21
<212>  DNA
<213>  Artificial Sequence

<220>
<223>  RAG-836

<400>  367
uggccaacaa agcugcugct t                                                21


<210>  368
<211>  21
<212>  DNA
<213>  Artificial Sequence

<220>

<223>  RAG-836

<400>  368
gcagcagcuu uguuggccat t                                                21


<210>  369
<211>  21
<212>  DNA
<213>  Artificial Sequence

<220>
<223>  RAG-837

<400>  369
cuggccaaca aagcugcugt t                                                21


<210>  370
<211>  21
<212>  DNA
<213>  Artificial Sequence

<220>
<223>  RAG-837

<400>  370
cagcagcuuu guuggccagt t                                                21


<210>  371
<211>  21
<212>  DNA
<213>  Artificial Sequence

<220>
<223>  RAG-838

<400>  371
ccuggccaac aaagcugcut t                                                21


<210>  372
<211>  21
<212>  DNA
<213>  Artificial Sequence

<220>
<223>  RAG-838

<400>  372
agcagcuuug uuggccaggt t                                                21


<210>  373
<211>  21
<212>  DNA
<213>  Artificial Sequence

<220>
<223>  RAG-840

<400>  373

uuccuggcca acaaagcugt t                                                21

<210>  374
<211>  21
<212>  DNA
<213>  Artificial Sequence

<220>
<223>  RAG-840

<400>  374
cagcuuuguu ggccaggaat t                                                21

<210>  375
<211>  21
<212>  DNA
<213>  Artificial Sequence

<220>
<223>  RAG-841

<400>  375
cuuccuggcc aacaaagcut t                                                21

<210>  376
<211>  21
<212>  DNA
<213>  Artificial Sequence

<220>
<223>  RAG-841

<400>  376
agcuuuguug gccaggaagt t                                                21

<210>  377
<211>  21
<212>  DNA
<213>  Artificial Sequence

<220>
<223>  RAG-843

<400>  377
cccuuccugg ccaacaaagt t                                                21

<210>  378
<211>  21
<212>  DNA
<213>  Artificial Sequence

<220>
<223>  RAG-843

<400>  378
cuuuguuggc caggaagggt t                                                21

<210>  379
<211>  21
<212>  DNA
<213>  Artificial Sequence

<220>
<223>  RAG-844

<400>  379
ccccuuccug gccaacaaat t                                                21


<210>  380
<211>  21
<212>  DNA
<213>  Artificial Sequence

<220>
<223>  RAG-844

<400>  380
uuuguuggcc aggaaggggt t                                                21


<210>  381
<211>  21
<212>  DNA
<213>  Artificial Sequence

<220>
<223>  RAG-845

<400>  381
uccccuuccu ggccaacaat t                                                21


<210>  382
<211>  21
<212>  DNA
<213>  Artificial Sequence

<220>
<223>  RAG-845

<400>  382
uuguuggcca ggaaggggat t                                                21


<210>  383
<211>  21
<212>  DNA
<213>  Artificial Sequence

<220>
<223>  RAG-846

<400>  383
cuccccuucc uggccaacat t                                                21


<210>  384
<211>  21
<212>  DNA

<213>  Artificial Sequence

<220>
<223>  RAG-846

<400>  384
uguuggccag gaaggggagt t                                                21


<210>  385
<211>  21
<212>  DNA
<213>  Artificial Sequence

<220>
<223>  RAG-848

<400>  385
uccuccccuu ccuggccaat t                                                21


<210>  386
<211>  21
<212>  DNA
<213>  Artificial Sequence

<220>
<223>  RAG-848

<400>  386
uuggccagga aggggaggat t                                                21


<210>  387
<211>  21
<212>  DNA
<213>  Artificial Sequence

<220>
<223>  RAG-849

<400>  387
auccuccccu uccuggccat t                                                21


<210>  388
<211>  21
<212>  DNA
<213>  Artificial Sequence

<220>
<223>  RAG-849

<400>  388
uggccaggaa ggggaggaut t                                                21


<210>  389
<211>  21
<212>  DNA
<213>  Artificial Sequence

<220>

<223>  RAG-853

<400>  389
ucaaauccuc cccuuccugt t                                                21


<210>  390
<211>  21
<212>  DNA
<213>  Artificial Sequence

<220>
<223>  RAG-853

<400>  390
caggaagggg aggauuugat t                                                21


<210>  391
<211>  21
<212>  DNA
<213>  Artificial Sequence

<220>
<223>  RAG-854

<400>  391
gucaaauccu ccccuuccut t                                                21


<210>  392
<211>  21
<212>  DNA
<213>  Artificial Sequence

<220>
<223>  RAG-854

<400>  392
aggaagggga ggauuugact t                                                21


<210>  393
<211>  21
<212>  DNA
<213>  Artificial Sequence

<220>
<223>  RAG-855

<400>  393
cgucaaaucc uccccuucct t                                                21


<210>  394
<211>  21
<212>  DNA
<213>  Artificial Sequence

<220>
<223>  RAG-855

<400>  394

ggaaggggag gauuugacgt t                                                21

<210>  395
<211>  21
<212>  DNA
<213>  Artificial Sequence

<220>
<223>  RAG-856

<400>  395
ucgucaaauc cuccccuuct t                                                21

<210>  396
<211>  21
<212>  DNA
<213>  Artificial Sequence

<220>
<223>  RAG-856

<400>  396
gaaggggagg auuugacgat t                                                21

<210>  397
<211>  21
<212>  DNA
<213>  Artificial Sequence

<220>
<223>  RAG-857

<400>  397
cucgucaaau ccuccccuut t                                                21

<210>  398
<211>  21
<212>  DNA
<213>  Artificial Sequence

<220>
<223>  RAG-857

<400>  398
aaggggagga uuugacgagt t                                                21

<210>  399
<211>  21
<212>  DNA
<213>  Artificial Sequence

<220>
<223>  RAG-858

<400>  399
acucgucaaa uccuccccut t                                                21

<210>  400
<211>  21
<212>  DNA
<213>  Artificial Sequence

<220>
<223>  RAG-858

<400>  400
aggggaggau uugacgagut t                                                21


<210>  401
<211>  21
<212>  DNA
<213>  Artificial Sequence

<220>
<223>  RAG-860

<400>  401
ucacucguca aauccuccct t                                                21


<210>  402
<211>  21
<212>  DNA
<213>  Artificial Sequence

<220>
<223>  RAG-860

<400>  402
gggaggauuu gacgagugat t                                                21


<210>  403
<211>  21
<212>  DNA
<213>  Artificial Sequence

<220>
<223>  RAG-861

<400>  403
cucacucguc aaauccucct t                                                21


<210>  404
<211>  21
<212>  DNA
<213>  Artificial Sequence

<220>
<223>  RAG-861

<400>  404
ggaggauuug acgagugagt t                                                21


<210>  405
<211>  21
<212>  DNA

<213>  Artificial Sequence

<220>
<223>  RAG-862

<400>  405
acucacucgu caaauccuct t                                                21


<210>  406
<211>  21
<212>  DNA
<213>  Artificial Sequence

<220>
<223>  RAG-862

<400>  406
gaggauuuga cgagugagut t                                                21


<210>  407
<211>  21
<212>  DNA
<213>  Artificial Sequence

<220>
<223>  RAG-864

<400>  407
caacucacuc gucaaaucct t                                                21


<210>  408
<211>  21
<212>  DNA
<213>  Artificial Sequence

<220>
<223>  RAG-864

<400>  408
ggauuugacg agugaguugt t                                                21


<210>  409
<211>  21
<212>  DNA
<213>  Artificial Sequence

<220>
<223>  RAG-865

<400>  409
acaacucacu cgucaaauct t                                                21


<210>  410
<211>  21
<212>  DNA
<213>  Artificial Sequence

<220>

<223>  RAG-865

<400>  410
gauuugacga gugaguugut t                                                21


<210>  411
<211>  21
<212>  DNA
<213>  Artificial Sequence

<220>
<223>  RAG-866

<400>  411
gacaacucac ucgucaaaut t                                                21


<210>  412
<211>  21
<212>  DNA
<213>  Artificial Sequence

<220>
<223>  RAG-866

<400>  412
auuugacgag ugaguuguct t                                                21


<210>  413
<211>  21
<212>  DNA
<213>  Artificial Sequence

<220>
<223>  RAG-867

<400>  413
agacaacuca cucgucaaat t                                                21


<210>  414
<211>  21
<212>  DNA
<213>  Artificial Sequence

<220>
<223>  RAG-867

<400>  414
uuugacgagu gaguugucut t                                                21


<210>  415
<211>  21
<212>  DNA
<213>  Artificial Sequence

<220>
<223>  RAG-868

<400>  415

cagacaacuc acucgucaat t                                                21

<210>  416
<211>  21
<212>  DNA
<213>  Artificial Sequence

<220>
<223>  RAG-868

<400>  416
uugacgagug aguugucugt t                                                21

<210>  417
<211>  21
<212>  DNA
<213>  Artificial Sequence

<220>
<223>  RAG-869

<400>  417
acagacaacu cacucgucat t                                                21

<210>  418
<211>  21
<212>  DNA
<213>  Artificial Sequence

<220>
<223>  RAG-869

<400>  418
ugacgaguga guugucugut t                                                21

<210>  419
<211>  21
<212>  DNA
<213>  Artificial Sequence

<220>
<223>  RAG-870

<400>  419
gacagacaac ucacucguct t                                                21

<210>  420
<211>  21
<212>  DNA
<213>  Artificial Sequence

<220>
<223>  RAG-870

<400>  420
gacgagugag uugucuguct t                                                21

<210>  421
<211>  21
<212>  DNA
<213>  Artificial Sequence

<220>
<223>  RAG-871

<400>  421
agacagacaa cucacucgut t                                                21


<210>  422
<211>  21
<212>  DNA
<213>  Artificial Sequence

<220>
<223>  RAG-871

<400>  422
acgagugagu ugucugucut t                                                21


<210>  423
<211>  21
<212>  DNA
<213>  Artificial Sequence

<220>
<223>  RAG-872

<400>  423
gagacagaca acucacucgt t                                                21


<210>  424
<211>  21
<212>  DNA
<213>  Artificial Sequence

<220>
<223>  RAG-872

<400>  424
cgagugaguu gucugucuct t                                                21


<210>  425
<211>  21
<212>  DNA
<213>  Artificial Sequence

<220>
<223>  RAG-873

<400>  425
ggagacagac aacucacuct t                                                21


<210>  426
<211>  21
<212>  DNA

<213>  Artificial Sequence

<220>
<223>  RAG-873

<400>  426
gagugaguug ucugucucct t                                                21


<210>  427
<211>  21
<212>  DNA
<213>  Artificial Sequence

<220>
<223>  RAG-874

<400>  427
aggagacaga caacucacut t                                                21


<210>  428
<211>  21
<212>  DNA
<213>  Artificial Sequence

<220>
<223>  RAG-874

<400>  428
agugaguugu cugucuccut t                                                21


<210>  429
<211>  21
<212>  DNA
<213>  Artificial Sequence

<220>
<223>  RAG-875

<400>  429
caggagacag acaacucact t                                                21


<210>  430
<211>  21
<212>  DNA
<213>  Artificial Sequence

<220>
<223>  RAG-875

<400>  430
gugaguuguc ugucuccugt t                                                21


<210>  431
<211>  21
<212>  DNA
<213>  Artificial Sequence

<220>

<223>  RAG-876

<400>  431
ucaggagaca gacaacucat t                                                21


<210>  432
<211>  21
<212>  DNA
<213>  Artificial Sequence

<220>
<223>  RAG-876

<400>  432
ugaguugucu gucuccugat t                                                21


<210>  433
<211>  21
<212>  DNA
<213>  Artificial Sequence

<220>
<223>  RAG-877

<400>  433
uucaggagac agacaacuct t                                                21


<210>  434
<211>  20
<212>  DNA
<213>  Artificial Sequence

<220>
<223>  RAG-877

<400>  434
gaguugucug ucuccugaat                                                  20


<210>  435
<211>  21
<212>  DNA
<213>  Artificial Sequence

<220>
<223>  RAG-878

<400>  435
auucaggaga cagacaacut t                                                21


<210>  436
<211>  21
<212>  DNA
<213>  Artificial Sequence

<220>
<223>  RAG-878

<400>  436

aguugucugu cuccugaaut t                                                21

<210>  437
<211>  21
<212>  DNA
<213>  Artificial Sequence

<220>
<223>  RAG-879

<400>  437
uauucaggag acagacaact t                                                21

<210>  438
<211>  21
<212>  DNA
<213>  Artificial Sequence

<220>
<223>  RAG-879

<400>  438
guugucuguc uccugaauat t                                                21

<210>  439
<211>  21
<212>  DNA
<213>  Artificial Sequence

<220>
<223>  RAG-880

<400>  439
guauucagga gacagacaat t                                                21

<210>  440
<211>  21
<212>  DNA
<213>  Artificial Sequence

<220>
<223>  RAG-880

<400>  440
uugucugucu ccugaauact t                                                21

<210>  441
<211>  21
<212>  DNA
<213>  Artificial Sequence

<220>
<223>  RAG-881

<400>  441
aguauucagg agacagacat t                                                21

<210>  442
<211>  21
<212>  DNA
<213>  Artificial Sequence

<220>
<223>  RAG-881

<400>  442
ugucugucuc cugaauacut t                                                21


<210>  443
<211>  21
<212>  DNA
<213>  Artificial Sequence

<220>
<223>  RAG-882

<400>  443
gaguauucag gagacagact t                                                21


<210>  444
<211>  21
<212>  DNA
<213>  Artificial Sequence

<220>
<223>  RAG-882

<400>  444
gucugucucc ugaauacuct t                                                21


<210>  445
<211>  21
<212>  DNA
<213>  Artificial Sequence

<220>
<223>  RAG-883

<400>  445
ggaguauuca ggagacagat t                                                21


<210>  446
<211>  21
<212>  DNA
<213>  Artificial Sequence

<220>
<223>  RAG-883

<400>  446
ucugucuccu gaauacucct t                                                21


<210>  447
<211>  21
<212>  DNA

<213>  Artificial Sequence

<220>
<223>  RAG-884

<400>  447
gggaguauuc aggagacagt t                                                21


<210>  448
<211>  21
<212>  DNA
<213>  Artificial Sequence

<220>
<223>  RAG-884

<400>  448
cugucuccug aauacuccct t                                                21


<210>  449
<211>  21
<212>  DNA
<213>  Artificial Sequence

<220>
<223>  RAG-885

<400>  449
ggggaguauu caggagacat t                                                21


<210>  450
<211>  21
<212>  DNA
<213>  Artificial Sequence

<220>
<223>  RAG-885

<400>  450
ugucuccuga auacucccct t                                                21


<210>  451
<211>  21
<212>  DNA
<213>  Artificial Sequence

<220>
<223>  RAG-886

<400>  451
uggggaguau ucaggagact t                                                21


<210>  452
<211>  21
<212>  DNA
<213>  Artificial Sequence

<220>

<223>  RAG-886

<400>  452
gucuccugaa uacuccccat t                                                21


<210>  453
<211>  21
<212>  DNA
<213>  Artificial Sequence

<220>
<223>  RAG-887

<400>  453
guggggagua uucaggagat t                                                21


<210>  454
<211>  21
<212>  DNA
<213>  Artificial Sequence

<220>
<223>  RAG-887

<400>  454
ucuccugaau acuccccact t                                                21


<210>  455
<211>  21
<212>  DNA
<213>  Artificial Sequence

<220>
<223>  RAG-888

<400>  455
uguggggagu auucaggagt t                                                21


<210>  456
<211>  21
<212>  DNA
<213>  Artificial Sequence

<220>
<223>  RAG-888

<400>  456
cuccugaaua cuccccacat t                                                21


<210>  457
<211>  21
<212>  DNA
<213>  Artificial Sequence

<220>
<223>  RAG-889

<400>  457

augugggag uauucaggat t                                                21

<210>  458
<211>  21
<212>  DNA
<213>  Artificial Sequence

<220>
<223>  RAG-889

<400>  458
uccugaauac uccccacaut t                                                21

<210>  459
<211>  21
<212>  DNA
<213>  Artificial Sequence

<220>
<223>  RAG-890

<400>  459
uaugugggga guauucaggt t                                                21

<210>  460
<211>  21
<212>  DNA
<213>  Artificial Sequence

<220>
<223>  RAG-890

<400>  460
ccugaauacu ccccacauat t                                                21

<210>  461
<211>  21
<212>  DNA
<213>  Artificial Sequence

<220>
<223>  RAG-891

<400>  461
cuaugugggg aguauucagt t                                                21

<210>  462
<211>  21
<212>  DNA
<213>  Artificial Sequence

<220>
<223>  RAG-891

<400>  462
cugaauacuc cccacauagt t                                                21

<210>  463
<211>  21
<212>  DNA
<213>  Artificial Sequence

<220>
<223>  RAG-892

<400>  463
gcuauguggg gaguauucat t                                                21


<210>  464
<211>  21
<212>  DNA
<213>  Artificial Sequence

<220>
<223>  RAG-892

<400>  464
ugaauacucc ccacauagct t                                                21


<210>  465
<211>  21
<212>  DNA
<213>  Artificial Sequence

<220>
<223>  RAG-893

<400>  465
ggcuaugugg ggaguauuct t                                                21


<210>  466
<211>  21
<212>  DNA
<213>  Artificial Sequence

<220>
<223>  RAG-893

<400>  466
gaauacuccc cacauagcct t                                                21


<210>  467
<211>  21
<212>  DNA
<213>  Artificial Sequence

<220>
<223>  RAG-894

<400>  467
gggcuaugug gggaguauut t                                                21


<210>  468
<211>  21
<212>  DNA

<213>  Artificial Sequence

<220>
<223>  RAG-894

<400>  468
aauacucccc acauagccct t                                                21

<